# EUROPEAN PATENT APPLICATION

(11) **EP 1 022 333 A1**
(43) Date of publication of application: **26.07.2000**
(21) Application number: 99119199.0
(22) Date of filing: 07.10.1999
(51) Int. Cl.: C12N 9/12, C12N 15/52, C12N 15/63, C12N 15/11, C12Q 1/68, C07K 16/40, G01N 33/53, A61P 35/00, A61P 9/00

(54) **Human tumor suppressor gene**

(30) Priority: 25.01.1999 JP 1622399
(71) Applicant: JCR PHARMACEUTICALS CO., LTD., Ashiya, Hyogo 659-0021 (JP)
(72) Inventor: Koga, Junichi, Kobe-shi Hyogo 651-2273 (JP); Kono, Keiko, Kobe-shi Hyogo 651-0061 (JP); Zolotaryov, Fyodor N., Nishi-ku Kobe-shi, Hyogo 651-2112 (JP)
(74) Representative: Behnisch, Werner, Dr.

(57) **Abstract**

A novel human tumor suppressor gene coding for a tumor suppressor protein and whose expression is inhibited by human growth hormone. The DNA composing the gene, and the protein coded by the gene are also provided. The DNA has a nucleotide sequence set forth under SEQ ID NO:1 or SEQ ID NO:2 in the Sequence Listing.

### Annex to the description

SEQUENCE LISTING
<110> JCR Pharmaceuticals Co., Ltd.

<120> Human Tumor Suppressing Gene

<160> 22

<210> 1
<211> 5486
<212> DNA
<213> Homo sapiens

<400> 1
cgcggcgtga gcgccccggg aagatggagc agtcgccgtc cacgccaccg ccgccgcccg     60

gggctccccc gtccctgcgg ggccagcagc agctccagcc accagtgccc ggtctcccgg    120

cgcgagaggc ccgggagccg ccggccagga cgcccccgag ggtgtagacc gcgcccctgg    180

agagagtgat aatcttcaaa atgaagactt tggaaaattt taggttctct ataggaacta    240

caaaaatgga aggaaagaac attttcaaaa ggaaattatt ttgaaagtat gtttacaaca    300

aactgatact attgacagtt ttttttttta aataataaaa cactttaaga agattgtatt    360

tatggtaaaa ggaaactgga ctaaca atg agg cca aag act ttt cct gcc acg    413
                             Met Arg Pro Lys Thr Phe Pro Ala Thr
                              1               5

act tat tct gga aat agc cgg cag cga ctg caa gag att cgt gag ggg    461
Thr Tyr Ser Gly Asn Ser Arg Gln Arg Leu Gln Glu Ile Arg Glu Gly
 10                  15                  20                  25

tta aag cag cca tcc aag tct tcg gtt cag ggg cta ccc gca gga cca    509

Leu Lys Gln Pro Ser Lys Ser Ser Val Gln Gly Leu Pro Ala Gly Pro
                30                  35                  40

aac agt gac act tcc ctg gat gcc aaa gtc ctg ggg agc aaa gat gcc 557
Asn Ser Asp Thr Ser Leu Asp Ala Lys Val Leu Gly Ser Lys Asp Ala
            45                  50                  55

acc agg cag cag cag cag atg aga gcc acc cca aag ttc gga cct tat 605
Thr Arg Gln Gln Gln Gln Met Arg Ala Thr Pro Lys Phe Gly Pro Tyr
        60                  65                  70

cag aaa gcc ttg agg gaa atc aga tat tcc ttg ttg cct ttt gct aat 653
Gln Lys Ala Leu Arg Glu Ile Arg Tyr Ser Leu Leu Pro Phe Ala Asn
    75                  80                  85

gaa tcg ggc acc tct gca gct gca gaa gtg aac cgg caa atg ctg cag 701
Glu Ser Gly Thr Ser Ala Ala Ala Glu Val Asn Arg Gln Met Leu Gln
90                  95                  100                 105

gaa ctg gtg aac gca gga tgc gac cag gag atg gct ggc cga gct ctc 749
Glu Leu Val Asn Ala Gly Cys Asp Gln Glu Met Ala Gly Arg Ala Leu
                110                 115                 120

aag cag act ggc agc agg agc atc gag gcc gcc ctg gag tac atc agc 797
Lys Gln Thr Gly Ser Arg Ser Ile Glu Ala Ala Leu Glu Tyr Ile Ser
            125                 130                 135

aag atg ggc tac ctg gac ccg agg aat gag cag att gtg cgg gtc att 845
Lys Met Gly Tyr Leu Asp Pro Arg Asn Glu Gln Ile Val Arg Val Ile
        140                 145                 150

aag cag acc tcc cca gga aag ggg ctc atg cca acc cca gtg acg cgg 893
Lys Gln Thr Ser Pro Gly Lys Gly Leu Met Pro Thr Pro Val Thr Arg
    155                 160                 165

agg ccc agc ttc gaa gga acc ggc gat tcg ttt gcg tcc tac cac cag 941
Arg Pro Ser Phe Glu Gly Thr Gly Asp Ser Phe Ala Ser Tyr His Gln
170                 175                 180                 185

ctg agc ggt acc ccc tac gag ggc cca agc ttc ggc gct gac ggc ccc 989
Leu Ser Gly Thr Pro Tyr Glu Gly Pro Ser Phe Gly Ala Asp Gly Pro
                190                 195                 200

acg gcg ctg gag gag atg ccg cgg ccg tac gtg gac tac ctt ttc ccc 1037
Thr Ala Leu Glu Glu Met Pro Arg Pro Tyr Val Asp Tyr Leu Phe Pro
            205                 210                 215

gga gtc ggc ccc cac ggg ccc ggc cac cag cac cag cac cca ccc aag 1085
Gly Val Gly Pro His Gly Pro Gly His Gln His Gln His Pro Pro Lys
        220                 225                 230

ggc tac ggt gcc agc gta gag gca gca ggg gca cac ttc ccg ctg cag 1133
Gly Tyr Gly Ala Ser Val Glu Ala Ala Gly Ala His Phe Pro Leu Gln
    235                 240                 245

ggc gcg cac tac ggg cgg ccg cac ctg ctg gtg cct ggg gaa ccc ctg 1181
Gly Ala His Tyr Gly Arg Pro His Leu Leu Val Pro Gly Glu Pro Leu
250                 255                 260                 265

ggc tac gga gtg cag cgc agc ccc tcc ttc cag agc aag acg ccg ccg 1229
Gly Tyr Gly Val Gln Arg Ser Pro Ser Phe Gln Ser Lys Thr Pro Pro
                270                 275                 280

gag acc ggg ggt tac gcc agc ctg ccc acg aag ggc cag gga gga ccg 1277
Glu Thr Gly Gly Tyr Ala Ser Leu Pro Thr Lys Gly Gln Gly Gly Pro
            285                 290                 295

cca ggc gcc ggc ctc gct ttc cca ccc cct gcc gcc ggg ctc tac gtg 1325

Pro Gly Ala Gly Leu Ala Phe Pro Pro Pro Ala Ala Gly Leu Tyr Val
        300                 305                 310

ccg cac cca cac cac aag cag gcc ggt ccc gcg gcc cac cag ctg cat      1373
Pro His Pro His His Lys Gln Ala Gly Pro Ala Ala His Gln Leu His
    315                 320                 325

gtg ctg ggc tcc cgc agc cag gtg ttc gcc agc gac agc ccc ccg cag      1421
Val Leu Gly Ser Arg Ser Gln Val Phe Ala Ser Asp Ser Pro Pro Gln
330                 335                 340                 345

agc ctg ctc act ccc tcg cgg aac agc ctc aac gtg gac ctg tat gaa      1469
Ser Leu Leu Thr Pro Ser Arg Asn Ser Leu Asn Val Asp Leu Tyr Glu
                350                 355                 360

ttg ggc agc acc tcc gtc cag cag tgg ccg gct gcc acc ctg gcc cgc      1517
Leu Gly Ser Thr Ser Val Gln Gln Trp Pro Ala Ala Thr Leu Ala Arg
            365                 370                 375

cgg gac tcc ctg cag aag ccg ggc ctg gag gcg ccg ccg cgc gcg cac      1565
Arg Asp Ser Leu Gln Lys Pro Gly Leu Glu Ala Pro Pro Arg Ala His
        380                 385                 390

gtg gcc ttc cgg cct gac tgc cca gtg ccc agc agg acc aac tcc ttc      1613
Val Ala Phe Arg Pro Asp Cys Pro Val Pro Ser Arg Thr Asn Ser Phe
    395                 400                 405

aac agc cac cag ccg cgg ccc ggt ccg cct ggc aag gcc gag ccc tcc      1661
Asn Ser His Gln Pro Arg Pro Gly Pro Pro Gly Lys Ala Glu Pro Ser
410                 415                 420                 425

ctg ccc gcc ccc aac acc gtg acg gct gtc acg gcc gcg cac atc ttg      1709
Leu Pro Ala Pro Asn Thr Val Thr Ala Val Thr Ala Ala His Ile Leu
                430                 435                 440

cac ccg gtg aag agc gtg cgt gtg ctg agg ccg gag ccg cag acg gct 1757
His Pro Val Lys Ser Val Arg Val Leu Arg Pro Glu Pro Gln Thr Ala
            445                 450                 455

gtg ggg ccc tcg cac ccc gcc tgg gtg ccc gcg cct gcc ccg gcc ccc 1805
Val Gly Pro Ser His Pro Ala Trp Val Pro Ala Pro Ala Pro Ala Pro
        460                 465                 470

gcc ccc gcc ccc gcc ccg gct gcg gag ggc ttg gac gcc aag gag gag 1853
Ala Pro Ala Pro Ala Pro Ala Ala Glu Gly Leu Asp Ala Lys Glu Glu
    475                 480                 485

cat gcc ctg gcg ctg ggc ggc gca ggc gcc ttc ccg ctg gac gtg gag 1901
His Ala Leu Ala Leu Gly Gly Ala Gly Ala Phe Pro Leu Asp Val Glu
490                 495                 500                 505

tac gga ggc cca gac cgg agg tgc ccg cct ccg ccc tac ccg aag cac 1949
Tyr Gly Gly Pro Asp Arg Arg Cys Pro Pro Pro Pro Tyr Pro Lys His
                510                 515                 520

ctg ctg ctg cgc agc aag tcg gag cag tac gac ctg gac agc ctg tgc 1997
Leu Leu Leu Arg Ser Lys Ser Glu Gln Tyr Asp Leu Asp Ser Leu Cys
            525                 530                 535

gca ggc atg gag cag agc ctc cgt gcg ggc ccc aac gag ccc gag ggc 2045
Ala Gly Met Glu Gln Ser Leu Arg Ala Gly Pro Asn Glu Pro Glu Gly
        540                 545                 550

ggc gac aag agc cgc aaa agc gcc aag ggg gac aaa ggc gga aag gat 2093
Gly Asp Lys Ser Arg Lys Ser Ala Lys Gly Asp Lys Gly Gly Lys Asp
    555                 560                 565

aaa aag cag att cag acc tct ccc gtt ccc gtc cgc aaa aac agc aga 2141

Lys Lys Gln Ile Gln Thr Ser Pro Val Pro Val Arg Lys Asn Ser Arg
570                 575                 580                 585

gac gaa gag aag aga gag tca cgc atc aag agc tac tcg cca tac gcc      2189
Asp Glu Glu Lys Arg Glu Ser Arg Ile Lys Ser Tyr Ser Pro Tyr Ala
                590                 595                 600

ttt aag ttc ttc atg gag cag cac gtg gag aat gtc atc aaa acc tac      2237
Phe Lys Phe Phe Met Glu Gln His Val Glu Asn Val Ile Lys Thr Tyr
            605                 610                 615

cag cag aag gtt aac cgg agg ctg cag ctg gag caa gaa atg gcc aaa      2285
Gln Gln Lys Val Asn Arg Arg Leu Gln Leu Glu Gln Glu Met Ala Lys
        620                 625                 630

gct gga ctc tgt gaa gct gag cag gag cag atg cgg aag atc ctc tac      2333
Ala Gly Leu Cys Glu Ala Glu Gln Glu Gln Met Arg Lys Ile Leu Tyr
    635                 640                 645

cag aaa gag tct aat tac aac agg tta aag agg gcc aag atg gac aag      2381
Gln Lys Glu Ser Asn Tyr Asn Arg Leu Lys Arg Ala Lys Met Asp Lys
650                 655                 660                 665

tct atg ttt gtc aag atc aaa acc ctg ggg atc ggt gcc ttt gga gaa      2429
Ser Met Phe Val Lys Ile Lys Thr Leu Gly Ile Gly Ala Phe Gly Glu
                670                 675                 680

gtg tgc ctt gct tgt aag gtg gac act cac gcc ctg tac gcc atg aag      2477
Val Cys Leu Ala Cys Lys Val Asp Thr His Ala Leu Tyr Ala Met Lys
            685                 690                 695

acc cta agg aaa aag gat gtc ctg aac cgg aat cag gtg gcc cac gtc      2525
Thr Leu Arg Lys Lys Asp Val Leu Asn Arg Asn Gln Val Ala His Val
        700                 705                 710

aag gcc gag agg gac atc ctg gcc gag gca gac aat gag tgg gtg gtc 2573
Lys Ala Glu Arg Asp Ile Leu Ala Glu Ala Asp Asn Glu Trp Val Val
    715                 720                 725

aaa ctc tac tac tcc ttc caa gac aaa gac agc ctg tac ttt gtg atg 2621
Lys Leu Tyr Tyr Ser Phe Gln Asp Lys Asp Ser Leu Tyr Phe Val Met
730                 735                 740                 745

gac tac atc cct ggt ggg gac atg atg agc ctg ctg atc cgg atg gag 2669
Asp Tyr Ile Pro Gly Gly Asp Met Met Ser Leu Leu Ile Arg Met Glu
                750                 755                 760

gtc ttc cct gag cac ctg gcc cgg ttc tac atc gca gag ctg act ttg 2717
Val Phe Pro Glu His Leu Ala Arg Phe Tyr Ile Ala Glu Leu Thr Leu
            765                 770                 775

gcc att gag agt gtc cac aag atg ggc ttc atc cac cga gac atc aag 2765
Ala Ile Glu Ser Val His Lys Met Gly Phe Ile His Arg Asp Ile Lys
        780                 785                 790

cct gat aac att ttg ata gat ctg gat ggt cac att aaa ctc aca gat 2813
Pro Asp Asn Ile Leu Ile Asp Leu Asp Gly His Ile Lys Leu Thr Asp
    795                 800                 805

ttc ggc ctc tgc act ggg ttc agg tgg act cac aat tcc aaa tat tac 2861
Phe Gly Leu Cys Thr Gly Phe Arg Trp Thr His Asn Ser Lys Tyr Tyr
810                 815                 820                 825

cag aaa ggg agc cat gtc aga cag gac agc atg gag ccc agc gac ctc 2909
Gln Lys Gly Ser His Val Arg Gln Asp Ser Met Glu Pro Ser Asp Leu
                830                 835                 840

tgg gat gat gtg tct aac tgt cgg tgt ggg gac agg ctg aag acc cta 2957

Trp Asp Asp Val Ser Asn Cys Arg Cys Gly Asp Arg Leu Lys Thr Leu
            845                 850                 855

gag cag agg gcg cgg aag cag cac cag agg tgc ctg gca cat tca ctg 3005
Glu Gln Arg Ala Arg Lys Gln His Gln Arg Cys Leu Ala His Ser Leu
        860                 865                 870

gtg ggg act cca aac tac atc gca ccc gag gtg ctc ctc cgc aaa ggg 3053
Val Gly Thr Pro Asn Tyr Ile Ala Pro Glu Val Leu Leu Arg Lys Gly
    875                 880                 885

tac act caa ctc tgt gac tgg tgg agt gtt gga gtg att ctc ttc gag 3101
Tyr Thr Gln Leu Cys Asp Trp Trp Ser Val Gly Val Ile Leu Phe Glu
890                 895                 900                 905

atg ctg gtg ggg cag ccg ccc ttt ttg gca cct act ccc aca gaa acc 3149
Met Leu Val Gly Gln Pro Pro Phe Leu Ala Pro Thr Pro Thr Glu Thr
                910                 915                 920

cag ctg aag gtg atc aac tgg gag aac acg ctc cac att cca gcc cag 3197
Gln Leu Lys Val Ile Asn Trp Glu Asn Thr Leu His Ile Pro Ala Gln
            925                 930                 935

gtg aag ctg agc cct gag gcc agg gac ctc atc acc aag ctg tgc tgc 3245
Val Lys Leu Ser Pro Glu Ala Arg Asp Leu Ile Thr Lys Leu Cys Cys
        940                 945                 950

tcc gca gac cac cgc ctg ggg cgg aat ggg gcc gat gac ctg aag gcc 3293
Ser Ala Asp His Arg Leu Gly Arg Asn Gly Ala Asp Asp Leu Lys Ala
    955                 960                 965

cac ccc ttc ttc agc gcc att gac ttc tcc agt gac atc cgg aag cag 3341
His Pro Phe Phe Ser Ala Ile Asp Phe Ser Ser Asp Ile Arg Lys Gln
970                 975                 980                 985

cca gcc ccc tac gtt ccc acc atc agc cac ccc atg gac acc tcg aat     3389
Pro Ala Pro Tyr Val Pro Thr Ile Ser His Pro Met Asp Thr Ser Asn
                990                 995                 1000

ttc gac ccc gta gat gaa gaa agc cct tgg aac gat gcc agc gaa ggt     3437
Phe Asp Pro Val Asp Glu Glu Ser Pro Trp Asn Asp Ala Ser Glu Gly
            1005                1010                1015

agc acc aag gcc tgg gac aca ctc acc tcg ccc aat aac aag cat cct     3485
Ser Thr Lys Ala Trp Asp Thr Leu Thr Ser Pro Asn Asn Lys His Pro
        1020                1025                1030

gag cac gca ttt tac gaa ttc acc ttc cga agg ttc ttt gat gac aat     3533
Glu His Ala Phe Tyr Glu Phe Thr Phe Arg Arg Phe Phe Asp Asp Asn
    1035                1040                1045

ggc tac ccc ttt cga tgc cca aag cct tca gga gca gaa gct tca cag     3581
Gly Tyr Pro Phe Arg Cys Pro Lys Pro Ser Gly Ala Glu Ala Ser Gln
1050                1055                1060                1065

gct gag agc tca gat tta gaa agc tct gat ctg gtg gat cag act gaa     3629
Ala Glu Ser Ser Asp Leu Glu Ser Ser Asp Leu Val Asp Gln Thr Glu
                1070                1075                1080

ggc tgc cag cct gtg tac gtg tagatggggg ccaggcaccc ccaccactcg        3680
Gly Cys Gln Pro Val Tyr Val
            1085

ctgcctccca ggtcagggtc ccggagccgg tgccctcaca ggccaatagg gaagccgagg   3740

gctgttttgt tttaaattag tccgtcgatt acttcacttg aaattctgct cttcaccaag   3800

aaaacccaaa caggacactt ttgaaaacag gactcagcat cgctttcaat aggcttttca   3860

ggaccttcac tgcattaaaa caatattttt gaaaatttag tacagtttag aaagagcact 3920

tattttgttt atatccattt tttcttacta aattataggg attaactttg acaaatcatg 3980

ctgctgttat tttctacatt tgtattttat ccatagcact tattcacatt taggaaaaga 4040

cataaaaact gaagaacatt gatgagaaat ctctgtgcaa taatgtaaaa aaaaaaaaga 4100

taacactctg ctcaatgtca cggagaccat tttatccaca caatggtttt tgttttttat 4160

tttttcccat gtttcaaaat tgtgatataa tgatataatg ttaaaagctg ctttttttgg 4220

ctttttgcat atctagtata ataggaagtg tgagcaaggt gatgatgtgg ctgtgatttc 4280

cgacgtctgg tgtgtggaga gtactgcatg agcagagttc ttctattata aaattaccat 4340

atcttgccat tcacagcagg tcctgtgaat acgtttttac tgagtgtctt taaatgaggt 4400

gttctagaca gtgtgctgat aatgtattgt gcgggtgacc tcttcgctat gattgtatct 4460

cttactgttt tgttaaagaa atgcagatgt gtaactgaga agtgatttgt gtgtgtgtct 4520

tggttgtgat tggattcttt gggggggggg gaactgaaac atttgtcata tactgaactt 4580

atatacatca aaagggatta atacagcgat gccaaaaagt ttaatcacgg acacacgtcc 4640

gtttctgtag tccgtatgct ctttcattct tggtagagct ggtatgtgga atgccatacc 4700

tctgacccta ctacttacct ttttactgac agactgccca cactgaaagc ttcagtgaat 4760

gttcttagtc ctgttttctt ctgttactgt caggaaactg agtgatctaa tggttctctc 4820

actttttttt tgttctttta gtgtactttg aagtatcaaa tcttaacttg gtttaaacaa 4880

tacatattcc taacctttgt aaaaaagcaa agattcttca aaatgacatt gaaataaaaa 4940

gtaagccata cgtattttct tagaagtata gatgtatgtg cgtgtataca cacacacaca 5000

cacacacaga gataaacaca atattcctta tttcaaatta gtatgattcc tatttaaagt 5060

gatttatatt tgagtaaaaa gttcaattct tttttgcttt ttaaaaaatc tgatgcttca 5120

taattttcat tatattattc cacatatttt tccttgaagt tcttagcata atgtatccat 5180

tacttagtat atatctaggc aacaacactt agaagtttat cagtgtttaa actaaaaaaa 5240

taaagattcc tgtgtactgg tttacatttg tgtgagtggc atactcaagt ctgctgtgcc 5300

tgtcgtcgtg actgtcagta ttctcgctat tttatagtcg tgccatgttg ttactcacag 5360

cgctctgaca tactttcatg tggtaggttc tttctcagga actcagttta actattattt 5420

attgatatat cattaccttt gaaaagcttc tactggcaca atttattatt aaaattttga 5480

atccag 5486

<210> 2
<211> 5486
<212> DNA
<213> Homo sapiens
<400> 2
cgcggcgtga gcgccccggg aagatggagc agtcgccgtc cacgccaccg ccgccgcccg 60
gggctccccc gtccctgcgg ggccagcagc agctccagcc accagtgccc ggtctcccgg 120
cgcgagaggc ccgggagccg ccggccagga cgcccccgag ggtgtagacc gcgcccctgg 180
agagagtgat aatcttcaaa atgaagactt tggaaaattt taggttctct ataggaacta 240
caaaaatgga aggaaagaac attttcaaaa ggaaattatt ttgaaagtat gtttacaaca 300
aactgatact attgacagtt ttttttttta aataataaaa cactttaaga agattgtatt 360
tatggtaaaa ggaaactgga ctaaca atg agg cca aag act ttt cct gcc acg 413
act tat tct gga aat agc cgg cag cga ctg caa gag att cgt gag ggg 461
tta aaa cag cca tcc aag tct tcg gtt cag ggg cta ccc gca gga cca 509
aac agt gac act tcc ctg gat gcc aaa gtc ctg ggg agc aaa gat gcc 557
acc agg cag cag cag cag atg aga gcc acc cca aag ttc gga cct tat 605
cag aaa gcc ttg agg gaa atc aga tat tcc ttg ttg cct ttt gct aat 653
gaa tcg ggc acc tct gca gct gca gaa gtg aac cgg caa atg ctg cag 701
gaa ctg gtg aac gca gga tgc gac cag gag atg gct ggc cga gct ctc 749
aag cag act ggc agc agg agc atc gag gcc gcc ctg gag tac atc agc 797
aag atg ggc tac ctg gac ccg agg aat gag cag att gtg cgg gtc att 845
aag cag acc tcc cca gga aag ggg ctc atg cca acc cca gtg acg cgg 893
agg ccc agc ttc gaa gga acc ggc gat tcg ttt gcg tcc tac cac cag 941
ctg agc ggt acc ccc tac gag ggc cca agc ttc ggc gct gac ggc ccc 989
acg gcg ctg gag gag atg ccg cgg ccg tac gtg gac tac ctt ttc ccc 1037
gga gtc ggc ccc cac ggg ccc ggc cac cag cac cag cac cca ccc aag 1085
ggc tac ggt gcc agc gta gag gca gca ggg gca cac ttc ccg ctg cag 1133
ggc gcg cac tac ggg cgg ccg cac ctg ctg gtg cct ggg gaa ccc ctg     1181
ggc tac gga gtg cag cgc agc ccc tcc ttc cag agc aag acg ccg ccg     1229
gag acc ggg ggt tac gcc agc ctg ccc acg aag ggc cag gga gga ccg     1277
cca ggc gcc ggc ctc gct ttc cca ccc cct gcc gcc ggg ctc tac gtg     1325
ccg cac cca cac cac aag cag gcc ggt ccc gtg gcc cac cag ctg cat     1373
gtg ctg ggc tcc cgc agc cag gtg ttc gcc agc gac agc ccc ccg cag     1421
agc ctg ctc act ccc tcg cgg aac agc ctc aac gtg gac ctg tat gaa     1469
ttg agc agc acc tcc gtc cag cag tgg ccg gct gcc acc ctg gcc cgc     1517
cgg gac tcc ctg cag aag ccg ggc ctg gag gcg ccg ccg cgc gcg cac 1565
gtg gcc ttc cgg cct gac tgc cca gtg ccc agc agg acc aac tcc ttc 1613
aac agc cac cag ccg cgg ccc ggt ccg cct ggc aag gcc gag ccc tcc 1661
ctg ccc gcc ccc aac acc gtg acg gct gtc acg gcc gcg cac atc ttg 1709
cac ccg gtg aag agc gtg cgt gtg ctg agg ccg gag ccg cag acg gct 1757
gtg ggg ccc tcg cac ccc gcc tgg gtg ccc gcg cct gcc ccg gcc ccc 1805
gcc ccc gcc ccc gcc ccg gct gcg gag ggc ttg gac gcc aag gag gag 1853
cat gcc ctg gcg ctg ggc ggc gca ggc gcc ttc ccg ctg gac gtg gag 1901
tac gga ggc cca gac cgg agg tgc ccg cct ccg ccc tac ccg aag cac 1949
ctg ctg ctg cgc agc aag tcg gag cag tac gac ctg gac agc ctg tgc 1997
gca ggc atg gag cag agc ctc cgt gcg ggc ccc aac gag ccc gag ggc 2045
ggc gac aag agc cgc aaa agc gcc aag ggg gac aaa ggc gga aag gat 2093
aaa aag cag att cag acc tct ccc gtt ccc gtc cgc aaa aac agc aga 2141
gac gaa gag aag aga gag tca cgc atc aag agc tac tcg cca tac gcc 2189
ttt aag ttc ttc atg gag cag cac gtg gag aat gtc atc aaa acc tac 2237
cag cag aag gtt aac cgg agg ctg cag ctg gag caa gaa atg gcc aaa 2285
gct gga ctc tgt gaa gct gag cag gag cag atg cgg aag atc ctc tac 2333
cag aaa gag tct aat tac aac agg tta aag agg gcc aag atg gac aag 2381
tct atg ttt gtc aag atc aaa acc ctg ggg atc ggt gcc ttt gga gaa 2429
gtg tgc ctt gct tgt aag gtg gac act cac gcc ctg tac gcc atg aag 2477
acc cta agg aaa aag gat gtc ctg aac cgg aat cag gtg gcc cac gtc 2525
aag gcc gag agg gac atc ctg gcc gag gca gac aat gag tgg gtg gtc 2573
aaa ctc tac tac tcc ttc caa gac aaa gac agc ctg tac ttt gtg atg 2621
gac tac atc cct ggt ggg gac atg atg agc ctg ctg atc cgg atg gag 2669
gtc ttc cct gag cac ctg gcc cgg ttc tac atc gca gag ctg act ttg 2717
gcc att gag agt gtc cac aag atg ggc ttc atc cac cga gac atc aag 2765
cct gat aac att ttg ata gat ctg gat ggt cac att aaa ctc aca gat 2813
ttc ggc ctc tgc act ggg ttc agg tgg act cac aat tcc aaa tat tac 2861
cag aaa ggg agc cat gtc aga cag gac agc atg gag ccc agc gac ctc 2909
tgg gat gat gtg tct aac tgt cgg tgt ggg gac agg ctg aag acc cta 2957
gag cag agg gcg cgg aag cag cac cag agg tgc ctg gca cat tca ctg 3005
gtg ggg act cca aac tac atc gca ccc gag gtg ctc ctc cgc aaa ggg 3053
tac act caa ctc tgt gac tgg tgg agt gtt gga gtg att ctc ttc gag 3101
atg ctg gtg ggg cag ccg ccc ttt ttg gca cct act ccc aca gaa acc 3149
cag ctg aag gtg atc aac tgg gag aac acg ctc cac att cca gcc cag 3197
gtg aag ctg agc cct gag gcc agg gac ctc atc acc aag ctg tgc tgc 3245
tcc gca gac cac cgc ctg ggg cgg aat ggg gcc gat gac ctg aag gcc 3293
cac ccc ttc ttc agc gcc att gac ttc tcc agt gac atc cgg aag cag 3341
cca gcc ccc tac gtt ccc acc atc agc cac ccc atg gac acc tcg aat 3389
ttc gac ccc gta gat gaa gaa agc cct tgg aac gat gcc agc gaa ggt 3437
agc acc aag gcc tgg gac aca ctc acc tcg ccc aat aac aag cat cct 3485
gag cac gca ttt tac gaa ttc acc ttc cga agg ttc ttt gat gac aat 3533
ggc tac ccc ttt cga tgc cca aag cct tca gga gca gaa gct tca cag 3581
gct gag agc tca gat tta gaa agc tct gat ctg gtg gat cag act gaa      3629
ggc tgc cag cct gtg tac gtg tagatggggg ccaggcaccc ccaccactcg      3680
ctgcctccca ggtcagggtc ccggagccgg tgccctcaca ggccaatagg gaagccgagg   3740
gctgttttgt tttaaattag tccgtcgatt acttcacttg aaattctgct cttcaccaag   3800
aaaacccaaa caggacactt ttgaaaacag gactcagcat cgctttcaat aggcttttca   3860
ggaccttcac tgcattaaaa caatattttt gaaaatttag tacagtttag aaagagcact   3920
tattttgttt atatccattt tttcttacta aattataggg attaactttg acaaatcatg   3980
ctgctgttat tttctacatt tgtattttat ccatagcact tattcacatt taggaaaaga   4040
cataaaaact gaagaacatt gatgagaaat ctctgtgcaa taatgtaaaa aaaaaaaaga   4100
taacactctg ctcaatgtca cggagaccat tttatccaca caatggtttt tgttttttat   4160
tttttcccat gtttcaaaat tgtgatataa tgatataatg ttaaaagctg ctttttttgg   4220
ctttttgcat atctagtata ataggaagtg tgagcaaggt gatgatgtgg ctgtgatttc   4280
cgacgtctgg tgtgtggaga gtactgcatg agcagagttc ttctattata aaattaccat   4340
atcttgccat tcacagcagg tcctgtgaat acgtttttac tgagtgtctt taaatgaggt   4400
gttctagaca gtgtgctgat aatgtattgt gcgggtgacc tcttcgctat gattgtatct 4460
cttactgttt tgttaaagaa atgcagatgt gtaactgaga agtgatttgt gtgtgtgtct 4520
tggttgtgat tggattcttt gggggggggg gaactgaaac atttgtcata tactgaactt 4580
atatacatca aaagggatta atacagcgat gccaaaaagt ttaatcacgg acacacgtcc 4640
gtttctgtag tccgtatgct ctttcattct tggtagagct ggtatgtgga atgccatacc 4700
tctgacccta ctacttacct ttttactgac agactgccca cactgaaagc ttcagtgaat 4760
gttcttagtc ctgttttctt ctgttactgt caggaaactg agtgatctaa tggttctctc 4820
actttttttt tgttctttta gtgtactttg aagtatcaaa tcttaacttg gtttaaacaa 4880
tacatattcc taacctttgt aaaaaagcaa agattcttca aaatgacatt gaaataaaaa 4940
gtaagccata cgtattttct tagaagtata gatgtatgtg cgtgtataca cacacacaca 5000
cacacacaga gataaacaca atattcctta tttcaaatta gtatgattcc tatttaaagt 5060
gatttatatt tgagtaaaaa gttcaattct tttttgcttt ttaaaaaatc tgatgcttca 5120
taattttcat tatattattc cacatatttt tccttgaagt tcttagcata atgtatccat 5180
tacttagtat atatctaggc aacaacactt agaagtttat cagtgtttaa actaaaaaaa 5240
taaagattcc tgtgtactgg tttacatttg tgtgagtggc atactcaagt ctgctgtgcc 5300
tgtcgtcgtg actgtcagta ttctcgctat tttatagtcg tgccatgttg ttactcacag 5360
cgctctgaca tactttcatg tggtaggttc tttctcagga actcagttta actattattt 5420
attgatatat cattaccttt gaaaagcttc tactggcaca atttattatt aaaattttga   5480
atccag                                                              5486

<210> 3
<211> 1088
<212> PRT
<213> Homo sapiens
<400> 3
Met Arg Pro Lys Thr Phe Pro Ala Thr Thr Tyr Ser Gly Asn Ser Arg
1               5                   10                  15

Gln Arg Leu Gln Glu Ile Arg Glu Gly Leu Lys Gln Pro Ser Lys Ser
            20                  25                  30

Ser Val Gln Gly Leu Pro Ala Gly Pro Asn Ser Asp Thr Ser Leu Asp
        35                  40                  45

Ala Lys Val Leu Gly Ser Lys Asp Ala Thr Arg Gln Gln Gln Gln Met
    50                  55                  60

Arg Ala Thr Pro Lys Phe Gly Pro Tyr Gln Lys Ala Leu Arg Glu Ile
65                  70                  75                  80

Arg Tyr Ser Leu Leu Pro Phe Ala Asn Glu Ser Gly Thr Ser Ala Ala
                85                  90                  95

Ala Glu Val Asn Arg Gln Met Leu Gln Glu Leu Val Asn Ala Gly Cys
            100                 105                 110

Asp Gln Glu Met Ala Gly Arg Ala Leu Lys Gln Thr Gly Ser Arg Ser
        115                 120                 125

Ile Glu Ala Ala Leu Glu Tyr Ile Ser Lys Met Gly Tyr Leu Asp Pro

    130                 135                 140

Arg Asn Glu Gln Ile Val Arg Val Ile Lys Gln Thr Ser Pro Gly Lys
145                 150                 155                 160

Gly Leu Met Pro Thr Pro Val Thr Arg Arg Pro Ser Phe Glu Gly Thr
                165                 170                 175

Gly Asp Ser Phe Ala Ser Tyr His Gln Leu Ser Gly Thr Pro Tyr Glu
            180                 185                 190

Gly Pro Ser Phe Gly Ala Asp Gly Pro Thr Ala Leu Glu Glu Met Pro
        195                 200                 205

Arg Pro Tyr Val Asp Tyr Leu Phe Pro Gly Val Gly Pro His Gly Pro
    210                 215                 220

Gly His Gln His Gln His Pro Pro Lys Gly Tyr Gly Ala Ser Val Glu
225                 230                 235                 240

Ala Ala Gly Ala His Phe Pro Leu Gln Gly Ala His Tyr Gly Arg Pro
                245                 250                 255

His Leu Leu Val Pro Gly Glu Pro Leu Gly Tyr Gly Val Gln Arg Ser
            260                 265                 270

Pro Ser Phe Gln Ser Lys Thr Pro Pro Glu Thr Gly Gly Tyr Ala Ser
        275                 280                 285

Leu Pro Thr Lys Gly Gln Gly Gly Pro Pro Gly Ala Gly Leu Ala Phe
    290                 295                 300

Pro Pro Pro Ala Ala Gly Leu Tyr Val Pro His Pro His His Lys Gln
305                 310                 315                 320

Ala Gly Pro Ala Ala His Gln Leu His Val Leu Gly Ser Arg Ser Gln
                325                 330                 335

Val Phe Ala Ser Asp Ser Pro Pro Gln Ser Leu Leu Thr Pro Ser Arg
            340                 345                 350

Asn Ser Leu Asn Val Asp Leu Tyr Glu Leu Gly Ser Thr Ser Val Gln
        355                 360                 365

Gln Trp Pro Ala Ala Thr Leu Ala Arg Arg Asp Ser Leu Gln Lys Pro
    370                 375                 380

Gly Leu Glu Ala Pro Pro Arg Ala His Val Ala Phe Arg Pro Asp Cys
385                 390                 395                 400

Pro Val Pro Ser Arg Thr Asn Ser Phe Asn Ser His Gln Pro Arg Pro
                405                 410                 415

Gly Pro Pro Gly Lys Ala Glu Pro Ser Leu Pro Ala Pro Asn Thr Val
            420                 425                 430

Thr Ala Val Thr Ala Ala His Ile Leu His Pro Val Lys Ser Val Arg
        435                 440                 445

Val Leu Arg Pro Glu Pro Gln Thr Ala Val Gly Pro Ser His Pro Ala
    450                 455                 460

Trp Val Pro Ala Pro Ala Pro Ala Pro Ala Pro Ala Pro Ala Pro Ala
465                 470                 475                 480

Ala Glu Gly Leu Asp Ala Lys Glu Glu His Ala Leu Ala Leu Gly Gly
                785                 490                 495

Ala Gly Ala Phe Pro Leu Asp Val Glu Tyr Gly Gly Pro Asp Arg Arg
            500                 505                 510

Cys Pro Pro Pro Pro Tyr Pro Lys His Leu Leu Leu Arg Ser Lys Ser
        515                 520                 525

Glu Gln Tyr Asp Leu Asp Ser Leu Cys Ala Gly Met Glu Gln Ser Leu
530 353 340

Arg Ala Gly Pro Asn Glu Pro Glu Gly Gly Asp Lys Ser Arg Lys Ser
545                 550                 555                 560

Ala Lys Gly Asp Lys Gly Gly Lys Asp Lys Lys Gln Ile Gln Thr Ser
                565                 570                 575

Pro Val Pro Val Arg Lys Asn Ser Arg Asp Glu Glu Lys Arg Glu Ser
            580                 585                 590

Arg Ile Lys Ser Tyr Ser Pro Tyr Ala Phe Lys Phe Phe Met Glu Gln
        595                 600                 605

His Val Glu Asn Val Ile Lys Thr Tyr Gln Gln Lys Val Asn Arg Arg
    610                 615                 620

Leu Gln Leu Glu Gln Glu Met Ala Lys Ala Gly Leu Cys Glu Ala Glu
625                 630                 635                 640

Gln Glu Gln Met Arg Lys Ile Leu Tyr Gln Lys Glu Ser Asn Tyr Asn
                645                 650                 655

Arg Leu Lys Arg Ala Lys Met Asp Lys Ser Met Phe Val Lys Ile Lys
            660                 665                 670

Thr Leu Gly Ile Gly Ala Phe Gly Glu Val Cys Leu Ala Cys Lys Val

        675                 680                 685

Asp Thr His Ala Leu Tyr Ala Met Lys Thr Leu Arg Lys Lys Asp Val
    690                 695                 700

Leu Asn Arg Asn Gln Val Ala His Val Lys Ala Glu Arg Asp Ile Leu
705                 710                 715                 720

Ala Glu Ala Asp Asn Glu Trp Val Val Lys Leu Tyr Tyr Ser Phe Gln
                725                 730                 735

Asp Lys Asp Ser Leu Tyr Phe Val Met Asp Tyr Ile Pro Gly Gly Asp
            740                 745                 750

Met Met Ser Leu Leu Ile Arg Met Glu Val Phe Pro Glu His Leu Ala
        755                 760                 765

Arg Phe Tyr Ile Ala Glu Leu Thr Leu Ala Ile Glu Ser Val His Lys
    770                 775                 780

Met Gly Phe Ile His Arg Asp Ile Lys Pro Asp Asn Ile Leu Ile Asp
785                 790                 795                 800

Leu Asp Gly His Ile Lys Leu Thr Asp Phe Gly Leu Cys Thr Gly Phe
                805                 810                 815

Arg Trp Thr His Asn Ser Lys Tyr Tyr Gln Lys Gly Ser His Val Arg
            820                 825                 830

Gln Asp Ser Met Glu Pro Ser Asp Leu Trp Asp Asp Val Ser Asn Cys
        835                 840                 845

Arg Cys Gly Asp Arg Leu Lys Thr Leu Glu Gln Arg Ala Arg Lys Gln
    850                 855                 860

His Gln Arg Cys Leu Ala His Ser Leu Val Gly Thr Pro Asn Tyr Ile
865                 870                 875                 880

Ala Pro Glu Val Leu Leu Arg Lys Gly Tyr Thr Gln Leu Cys Asp Trp
                885                 890                 895

Trp Ser Val Gly Val Ile Leu Phe Glu Met Leu Val Gly Gln Pro Pro
            900                 905                 910

Phe Leu Ala Pro Thr Pro Thr Glu Thr Gln Leu Lys Val Ile Asn Trp
        915                 920                 925

Glu Asn Thr Leu His Ile Pro Ala Gln Val Lys Leu Ser Pro Glu Ala
    930                 935                 940

Arg Asp Leu Ile Thr Lys Leu Cys Cys Ser Ala Asp His Arg Leu Gly
945                 950                 955                 960

Arg Asn Gly Ala Asp Asp Leu Lys Ala His Pro Phe Phe Ser Ala Ile
                965                 970                 975

Asp Phe Ser Ser Asp Ile Arg Lys Gln Pro Ala Pro Tyr Val Pro Thr
            980                 985                 990

Ile Ser His Pro Met Asp Thr Ser Asn Phe Asp Pro Val Asp Glu Glu
        995                 1000                1005

Ser Pro Trp Asn Asp Ala Ser Glu Gly Ser Thr Lys Ala Trp Asp Thr
    1010                1015                1020

Leu Thr Ser Pro Asn Asn Lys His Pro Glu His Ala Phe Tyr Glu Phe
1025                1030                1035                1040

Thr Phe Arg Arg Phe Phe Asp Asp Asn Gly Tyr Pro Phe Arg Cys Pro
                1045                1050                1055

Lys Pro Ser Gly Ala Glu Ala Ser Gln Ala Glu Ser Ser Asp Leu Glu
            1060                1065                1070

Ser Ser Asp Leu Val Asp Gln Thr Glu Gly Cys Gln Pro Val Tyr Val
        1075                1080                1085

<210> 4
<211> 1088
<212> PRT
<213> Homo sapiens
<400> 4

<210> 5
<211> 17
<212> DNA
<213> M13 phage

<400> 5
gtaaaacgac ggccagt                                                17

<210> 6
<211> 17
<212> DNA
<213> M13 phage

<400> 6
caggaaacag ctatgac                                                17

<210> 7
<211> 26
<212> DNA
<213> Homo sapiens

<400> 7

tccaaatatt accagaaagg gagcca 26

<210> 8

<211> 19

<212> DNA

<213> Homo sapiens

<400> 8

gacctctggg atgatgtgt 19

<210> 9

<211> 26

<212> DNA

<213> Homo sapiens

<400> 9

agaggtcttg ggcacatttc actggt 26

<210> 10

<211> 19

<212> DNA

<213> Homo sapiens

<400> 10

cctgagcacg cattttacg 19

<210> 11

<211> 19

<212> DNA

<213> Homo sapiens

<400> 11

acaatggcta cccctttcg 19

<210> 12
<211> 18
<212> DNA
<213> Homo sapiens

<400> 12
ttcgtaaaat gcgtgctc 18

<210> 13
<211> 20
<212> DNA
<213> Homo sapiens

<400> 13
cctgtttggg ttttcttggt 20

<210> 14
<211> 28
<212> DNA
<213> Homo sapiens

<400> 14
tcatcacctt gctcacactt ccctatta 28

<210> 15
<211> 21
<212> DNA
<213> Homo sapiens

<400> 15
catcaccttg ctcacacttc c 21

<210> 16

<211> 25
<212> DNA
<213> Homo sapiens

<400> 16
gtcggaaatc acagccacat catca 25

<210> 17
<211> 19
<212> DNA
<213> Homo sapiens

<400> 17
ttcgtttgcg tcctaccac 19

<210> 18
<211> 16
<212> DNA
<213> Homo sapiens

<400> 18
gcggcgtctt gctctg 16

<210> 19
<211> 21
<212> DNA
<213> Homo sapiens

<400> 19
ctgctctggt ctcaatttaa g 21

<210> 20
<211> 19
<212> DNA

<213> Homo sapiens

<400> 20
caagtctgct gtgcctgtc 19

<210> 21
<211> 22
<212> DNA
<213> T7 phage

<400> 21
ctaatacgac tcactatagg ga 22

<210> 22
<211> 20
<212> DNA
<213> T7 phage

<400> 22
ggttttccca gtcacgacgt 20

## Description

### FIELD OF THE INVENTION

The present invention relates to a human tumor suppressor gene whose expression is inhibited by treatment with a growth factor, the DNA composing the gene, and the protein coded by the DNA.

### BACKGROUND OF THE INVENTION

Due to its growth stimulating effect, recombinant growth hormone (rhGH) has been widely used in the world as a therapeutic drug for children with GH secretion deficiency, Turner syndrome or chronic nephropathy [Neely E.K., and Rosenfeld R.G., Ann. Rev. Med., 45:407-420 (1994)]. In addition, as it exhibits a variety of physiological effects, expanded pharmaceutical use of GH is expected in connection with, for example, lipid metabolism, protein anabolism, osteoporosis and hemopoiesis as well as, more widely, with aging.

The higher animals has some 100,000 genes, and about 15 % of them are expressed in cells and govern all the processes of activity of life, i.e., development, proliferation, differentiation, homeostasis, regulation of cell cycle, etc. Regulation of protein synthesis by expression of these genes is performed primarily at the levels of transcription (mRNA) and translation, and GH is considered to be closely involved in those functions.

On the other hand, through research of tumors, which are caused by abnormal proliferation of cells, existence of a tumor gene was suggested in 1970's, and then Hanabusa et al. for the first time identified a tumor gene v-src. Afterwards, through analyses of hereditary tumors, a variety of tumor suppressor genes were found as genes (inhibition of) which are causative of tumors, and proofs also have been accumulated indicating that progression of certain tumors are suppressed by tumor suppressor genes, which suppress cell proliferation.

In general determination of a tumor suppressor gene, the criteria are (1) that point mutation and deletion are found in both of the allelic genes in the tumor, and (2) that a mutation is found in germ cells, which contain one of allelic genes, in a cancer family with a high incidence of hereditary tumor. The characteristic of a tumor suppressor gene consists in that the loss of its function leads to carcinogenesis.

It has been revealed that, in the development and progression of cancer, a series of gene mutations are involved including activation of a tumor gene and inactivation of a tumor suppressor gene due to its deletion or mutation [Schmandt, r. et al. Clin. Chem., 39: 2375-2385 (1993)]. Physiological importance of tumor suppressor genes has been recognized through identification of tumor suppressor genes and accumulation of proofs indicating that lack of normal allele in many of tumor cells [Knudson, A.G. et al., Proc. Natl. Acad. Sci. 90: 10914-10921 (1993)].

More than ten primary tumor suppressor genes have been found so far, including RB1 gene, whose mutation is causative of retinoblastoma [Friend, S.H. et al., Proc. Natl. Acad. Sci. USA, 84: 9059-9063 (1987)], p53 gene causative of colorectal cancer [Lane, D.P. et al., Nature, 278: 261-263 (1979)], and WTI gene causative of Wilms tumor [Call, K.M. et al., Cell, 60: 509-520 (1990)].

Recently, many researches have been conducted to make clear the relation between certain types of tumors and deletion from chromosome 13q. With the help of different polymorphic DNA markers, restriction fragment length polymorphism (RFLP) has enabled detection of deletion of an allelic gene, i.e. loss of heterozygosity (LOH) in a specific region of a chromosome.

Deletion of an allelic gene on chromosome 13q12 was observed in tumors of the prostate, ovary, cervix, colon, ureter, and mammary gland [Gudmundsson, J. et al., Cancer Res., 55: 4830-4832 (1995)]. Two cancer susceptibility genes, BRCA1 on chromosome 17q21 and BRCA2 on chromosome 13q12, for example, are thought to be responsible for approximately 80 % of breast cancer cases in families with multiple cases of early-onset female breast cancer in the United States [Gayther, S.A. et al., Mol. Med.-Today, 3: 168-174 (1997)]. However, the figure has been obtained just for American population and is expected to vary for other nations. For example, in Finnish breast cancer families, altogether only 21 % were accounted for by mutations in BRCA1 and BRCA2 genes [Verhmanen, P. et al., Hum. Mol. Genet., 6: 2309-2315 (1997)]. These data indicate that some additional breast and breast-ovarian cancer susceptibility genes are likely to be important, and some of them are anticipated on chromosome 13q12-13q13 [Van Den Berg J., et al., Br. J. Cancer, 74: 1615-1619 (1996)].

Deletions from chromosome 13, including chromosome 13q12, were found in many types of human lung cancers such as small cell carcinoma [Yokota, J. et al., Proc. Natl. Acad. Sci. USA, 84: 9252-9256 (1987)], squamous cell carcinoma, large cell carcinoma and adenocarcinoma [Weston, A. et al., Proc. Natl. Acad. Sci. USA., 86: 5099-5103 (1989)]. However, since extended regions of chromosome 13 were lost, it was impossible to pin-point the culprit locus. Recently, much more accurate data became available for non-small cell lung carcinoma showing a specific loss of 13q12 [Virmani, A.K. et al, Genes Chromosomes Cancer, 21: 308-319 (1998)]. Moreover, some unknown tumor suppressor gene are expected to exist in this region [Tamura, K. et al., Int. J. Cancer, 74: 45-49 (1997)].

Distinct deletion of chromosome 13q12 locus was found in 67 % of hepatocellular carcinoma (regardless of being positive/negative with regard to hepatitis B virus) [Jacob, A.N. et al., Oncogene, 13: 213-221 (1996), Walker, G.J. et al., Cancer Res., 51: 4367-4370 (1991): 4367-4370 (1991)]; in head and neck squamous cell carcinoma (believed to be due to the deletion of other, as yet unidentified tumor suppressor gene(s) localized to chromosome 13q, particularly to 13q12 [Kirkpatrick, H. et al., Oncogene, 14: 2189-2193 (1997)]; in pancreatic carcinoma [Schutte, M. et al., Proc. Natl. Acad. Sci. USA, 92: 5950-5954 (1995)] and adenocarcinoma [Jacob, A.N. et al., Oncogene, 13: 213-221 (1996)]; in pituitary adenoma (reflecting aggressive biological activity with potential value as a prognostic marker) [Pearce, S.H. et al., Clin. Endocrinol. (Oxf.), 45: 195-200 (1996), Bates, A.S. et al., J. Clin. Endocrinol. Metab., 82: 818-824 (1997)]; in parathyroid adenoma [Pearce, S.H. et al., Clin. Endocrinol. (Oxf.), 45: 195-200 (1996), Yoshimoto, K. et al., Clin. Endocrinol. (Oxf.), 48: 67-72 (1998)]; and in lipoma [Mandahl, N. et al., J. Cancer Res. Clin. Oncol., 120: 707-711 (1994), Sreekantaiah, C. et al., Cancer Genet. Cytogenet., 39: 281-288 (1989)].

Extensive data associated with the loss of chromosome 13q12 were obtained for some blood cancers: multiple myeloma and plasma cell leukemia [Avet-Loiseau, H. et al., Genes Chromosomes Cancer, 19: 124-133 (1997)], myeloproliferative neoplasms [Still, I.H. et al., Ann. Hum.-Genet., 61(Ptl): 15-24 (1997)] and nonspecific myeloproliferative disorder associated with T-cell leukemia/lymphoma and peripheral blood eosinophilia [Still, I.H. et al., Blood, 90: 3136-3141 (1997)], chronic lymphocytic leukemia [Garcia-Marco, J.A. et al., Cancer Genet. Cytogenet., 94: 52-58 (1997), Garcia-Marco, J.A. et al., Blood, 88: 1568-1575 (1996), Catovsky, D., Hematol. Cell Ther., 39 Suppl. 1: S5-S11 (1997), Garcia-Marco, J.A. et al., Br. J. Haematol., 99: 708-709 (1997)]. Some of these articles predicted new tumor suppressor gene(s) localized to chromosome 13ql2 [Garcia-Marco, J.A. et al., Cancer Genet. Cytogenet., 94: 52-58 (1997), Garcia-Marco, J.A. et al., Blood, 88: 1568-1575 (1996), Catovsky, D., Hematol. Cell Ther., 39 Suppl. 1: S5-S11 (1997)].

Such tumor suppressor genes are expected to be conserved among biological species, taking part in the regulation of cell proliferation and apoptosis, with many products of the tumor suppressor genes playing roles in the regulation of cell cycle. It has thus been revealed that tumor suppressor gene products are associated with cell adhesion proteins, signal introduction molecules, transcription factors, cell cycle regulating molecules or apoptosis regulating molecules.

On the other hand, recent gene analyses revealed that approximately 80 % of human genes are homologous to Drosophila melanogaster, and Drosophila is expected to be an excellent model in studying human tumor development at the molecular level [Cookson, C., Financial Times, June 14 (1997)]. The number of genes comprising the genome of Drosophila is about 10,000. Although this number is smaller than the number of genes comprising human genome, i.e. about 100,000, human genes are considered to be similar to the insect genes, since most of the human genes are multiplicate and derived genes. Many proteins and their domains as well as whole complexes and a variety of metabolic and signal transduction systems are conserved between human and Drosophila [Artavanis-Tsakonas S. et al, Science, 268: 225-232 (1995)].

In addition, it has also been found that Drosophila melanogaster develops tumors with common characteristics to the human tumors and that tumor genes and tumor suppressor genes of Drosophila melanogaster are homologous to tumor genes and tumor suppressor genes of human [Miklos, G.L.G. and Rubin, G.M., Cell, 86: 521-529 (1996)]. An approach utilizing gene data for Drosophila melanogaster, therefore, provides a powerful tool in the search for a novel gene [Tian Xu, et al., Developments, 121: 1053-1063 (1995)].

Upon the above background, the objective of the present invention is to provide a novel human tumor suppressor gene whose expression is inhibited by human growth hormone and the like, the DNA composing the gene, and the protein coded by the gene.

### SUMMARY OF THE INVENTION

As will be stated later, in a research using rabbit's chondrocytes, the present inventors found that the expression of certain gene was inhibited by growth factors such as growth hormone (GH), and examined the gene after isolation and identification. Upon the results, the inventors reached a conclusion that the gene was likely to be a novel tumor suppressor gene, and thus attained the present invention.

Thus, the present invention provides a DNA having a nucleotide sequence set forth under SEQ ID NO:1 or SEQ ID NO:2 in the Sequence Listing.

In addition, the present invention provides a DNA composing a human gene whose expression is inhibited by a growth factor and which has a nucleotide sequence set forth under SEQ ID NO:1 or SEQ ID NO:2 in the Sequence Listing or a nucleotide sequence with deletion, substitution or addition of one or more bases relative to either of said nucleotide sequences.

Further, the present invention provides the DNA composing said human gene, wherein the growth factor is human growth hormone or insulin-like growth factor-1.

Furthermore, the present invention provides a DNA composing a human gene coding for a tumor suppressor protein which has a nucleotide sequence set forth under SEQ ID NO:1 or SEQ ID NO:2 in the Sequence Listing or a nucleotide sequence with deletion, substitution or addition of one or more bases relative to either of said nucleotide sequences.

Furthermore, the present invention provides a protein having an amino acid sequence set forth under SEQ ID NO:3 or SEQ ID NO:4 in the Sequence Listing.

Furthermore, the present invention provides a protein whose expression is inhibited by a growth factor and which has an amino acid sequence set forth under SEQ ID NO:3 or SEQ ID NO:4 in the Sequence Listing or an amino acid sequence with deletion, substitution or addition of one or more amino acids relative to either of said amino acid sequences.

Furthermore, the present invention provides the protein referred to above wherein the growth factor is human growth hormone or insulin-like growth factor-1.

Furthermore, the present invention provides a tumor suppressor protein having an amino acid sequence set forth under SEQ ID NO:3 or SEQ ID NO:4 in the Sequence Listing or an amino acid sequence with deletion, substitution or addition of one or more amino acids relative to either of said amino acid sequences.

Furthermore, the present invention provides a DNA comprising a nucleotide sequence coding a protein having one of the aforementioned amino acid sequences.

Furthermore, the present invention provides a probe comprising a DNA which hybridizes with a DNA consisting of one of the aforementioned nucleotide sequences.

Furthermore, the present invention provides a recombinant vector including a DNA consisting of one of the aforementioned nucleotide sequences.

Furthermore, the present invention provides a DNA fragment for use as a primer and consisting of a partial sequence of one of the aforementioned nucleotide sequences.

Furthermore, the present invention provides a diagnostic pharmaceutical preparation for human use comprising the aforementioned probe. The diagnostic pharmaceutical preparation is useful, in dwarfism, gigantism, acromegaly, angiopathy, diabetic nephropathy or cardiopathy or in malignant tumor including leukemia, for examining expression of a tumor suppressor gene which is inhibited by a growth factor.

Furthermore, the present invention provides an antineoplastic pharmaceutical preparation containing one of the aforementioned proteins.

Furthermore, the present invention provides a polyclonal or monoclonal antibody against one of the aforementioned proteins.

Furthermore, the present invention provides a diagnostic pharmaceutical preparation containing one of the aforementioned antibodies for examining expression of a tumor suppressor gene.

Thus, the DNAs composing the gene of the present invention, their transcripts mRNAs, and their translated proteins may be used as therapeutic, diagnostic and prophylactic drugs for cardiopathy, angiopathy, diabetic nephropathy and malignant tumors such as breast cancer, renal adenocarcinoma, colorectal cancer and leukemia, which could be induced by elevated levels of growth hormone or IGF-1 occurring in such situations as excessive secretion of growth hormone in gigantism/acromegaly or excessive dosage of growth hormone in dwarfism.

### DETAILED DESCRIPTION OF THE INVENTION

An analysis was conducted on LOH in samples of 84 primary tumors from sporadic, familial and hereditary breast cancers. As a result, LOH at the BRCA2 (breast cancer susceptibility gene) region was found in 34 % of the tumor samples and LOH at the RB1 region in 27 %. However, selective LOH at BRCA2 occurred only in 7 % of the tumors, whereas selective LOH at RB1 also was observed only in 7 %.

Moreover, a few of the tumors demonstrated a restricted deletion pattern suggesting the presence of additional tumor-suppressor genes both proximal and distal of BRCA2. A highly significant and independent correlation was also revealed between LOH at BRCA2 and early recurrence and death. The results suggest that inactivation of one or more tumor-suppressor genes in the 13q12-13q13 region confers strong potential toward growth and results in poor prognosis in both familial and sporadic breast cancers.

Using mRNA differential display and PCR-select cDNA subtraction, the present inventors preliminarily attempted a detection of genes regulated by growth hormone, and thereby demonstrated the existence of genes which were regulated by growth hormone in rabbit chondrocytes. Thus, using mRNA differential display, the electrophoretic band N 119 was found to be inhibited by treatment with growth hormone. This band was excised, and DNA contained in it was extracted, cloned and sequenced. The DNA fragment was found to be only 219 bases long, and no meaningful homology to known genes or EST (Expressed Sequence Tags) was observed. The DNA corresponding to the band N 119 was homologous to the DNAs corresponding to the bands N 62, 71, 111 and 112. So, the DNAs from these five bands were assigned to a homology cluster HCD10, and the rabbit gene of interest was designated HCD10(119). In addition to the finding of inhibition by growth hormone, also found was that the transcription of HCD10(119) gene was activated by concanavalin A.

The 5'-RACE (Rapid Amplification of 5'-cDNA Ends) method was employed for cloning of some part of the gene coding the HCD10(119) protein. DNA of 2.5 kb fragment of the gene was sequenced and found to be homologous to tumor suppressor gene warts of Drosophila melanogaster.

Furthermore, in the present study, it was found in an experiment using rabbits chondrocytes that the expression of certain gene was inhibited by growth hormone (GH). Based on these data, the present inventors predicted the existence of a tumor suppressor gene whose expression is inhibited by GH (GHITSG: Growth Hormone Inhibited Tumor Suppressor Gene), and continued investigation.

Then, in BLAST search using the 2.5 kb DNA sequence of the rabbit HCD10(119) gene fragment, the present inventors found some human ESTs and compiled them into a conjectural 1.65 kb region of a human analog (hGHITS: Human Growth Hormone Inhibited Tumor Suppressor Gene) using DNASIS (Hitachi Software Engineering Co., Ltd.). Unfortunately, it was not possible to get a reliable sequence of the gene by such a method. It was because that ESTs were basically rather inaccurate [Sudo, K. et al., Genomics, 24: 276-279 (1994), Frigerio, J.-M., Hum. Mol. Genet. 4(1): 37-43 (1995), Lanfranchi, G. et al, Genome Research, 6: 35-42 (1996)]. Then, sequencing using a group of primers was attempted.

Five forward and five reverse primers covering the 1.65 kb human DNA fragment were designed with OLIGO (a software for analysis of primer: purchased from National Bioscience, Inc.), and seven primer sets were employed in PCRs using a Universal cDNA library as a template:
1) H119U142 + H119L739,
2) H119U142 + H119L1048,
3) H119U142 + H119L1499,
4) H119U736 + H119L1048,
5) H119U736 + H119L1499,
6) H119U782 + H119L1048, and
7) H119U782 + H119L1499

Eventually, the primer sets 1) and 7) were selected, since they produced DNA bands of apparently proper sizes which would be the most suitable for use as hybridization probes.

PCRs were repeated with primer sets 1) and 7) and with a panel of 14 separate cDNA libraries as templates. According to the results obtained, the Human Lung cDNA Library (λgt10) was selected, for its amplification gave strong DNA bands with either pair of primers.

The DNA bands thus obtained were cloned in a plasmid vector (pMOSBlue: Amersham), and since DNA sequencing confirmed their authenticity, the specific hybridization probes were produced by growing the E. coli strains carrying respective plasmids, purifying the plasmid DNAs and subjecting them to digestion with EcoRI and SalI restriction enzymes, separating the DNA fragments by electrophoresis, and purifying the DNA insertions.

Seventeen phage clones carrying various regions of hGHITS gene were recovered. Their DNAs were purified, recloned into a plasmid vector (pUC18: Pharmacia) and sequenced. A 4892 base nucleotide sequence was reconstructed, but it lacked both 5'- and 3'-ends of the full length gene.

Using OLIGO, primers pairs were designed for both ends of the nucleotide sequence: the primer pair of H3111U13 and H311L320 for 5'-region, and the primer pair of H316U39 and H319L498 for 3'-region of the gene. Amplification of cloned phage DNA fragments with these primers gave PCR products that were separated by agarose gel electrophoresis. They were then directly used as probes without cloning. Thirteen phage clones hybridizing with the 5'-probe and twelve phage clones hybridizing with the 3'-probe were recovered and analyzed.

Analysis of these phage clones revealed the existence of two presumably functional alleles. The full-length 5486 base nucleotide sequence, hGHITS1 (SEQ ID NO:1 in the Sequence Listing) and hGHITS2 (SEQ ID NO:2) were assembled. And respective proteins consisting of the sequence of 1088 amino acid residues corresponding to the respective coding regions of hGHITS1 and hGHITS2 were deduced (SEQ ID NO:3 and SEQ ID NO: 4). Three differences were found in the protein coding regions of these nucleotide sequences: hGHITS1 allele carries "g" at nucleotide position 467 (thereby, amino acid 27 was Lys), "c" at nucleotide position 1357 (thereby, amino acid 324 was Ala), and "g" at the nucleotide position 1473 (thus, amino acid 363 was Gly), while hGHITS2 allele carries "a" at nucleotide position 467 (no amino acid change), "t" at nucleotide position 1357 (amino acid 324 was Val), and "a" at nucleotide position 1473 (amino acid 363 was Ser). Between these sequences with the three differences, assignment to normal/mutant types has so far not been established.

According to PSORT analysis, it is most likely that the protein is an enzyme and localized to nucleus, with a possible cleavage site between amino acids 60 and 61.

Using BLASTP search, the amino acid sequences of hGHITS protein were compared with known polypeptide sequences . The Drosophila tumor suppressor gene warts [Justice R.W. et al., Genes & Dev., 9: 534-546 (1995)] (also called gene "lats": Xu, T. et al., Development, 121: 1053-1063 (1995)) was revealed to have the highest homology with hGHITS gene. The alignment of hGHITS versus warts gene using "GeneStream align" clearly showed a remarkable homology at C-end of the enzymes. MOTIF search unveiled 8 motifs including serine/threonine protein kinase active-site signature and protein kinase ATP-binding region signature situated at the region with high homology to Drosophila warts gene, which is also a protein kinase.

It is well known that protein kinase catalytic domains with limited divergence from other member of the family can be expected to play a similar role in cellular physiology [Hanks, S.K. et al., Science 241: 42-52 (1988)]. On the other hand, human homologues of Drosophila melanogaster tumor suppressors have been isolated and shown to be functionally conserved [Maie, A.R. St. J. & Xu, T., Am. J. Hum. Genet., 61: 1006-1010 (1997)]. Tumor suppressor gene warts of Drosophila melanogaster is essential to the normal growth of cells as well as the maintenance of their normal morphology. This gene is considered to be a tumor suppressor gene as its loss was found to cause abnormal proliferation, and it resembles human muscular dystrophy kinase. These facts provide a strong support to that hGHITS be a tumor suppressor gene.

The hGHITS gene showed the highest homology with 1053 bp long EST called DRES 7. DRES 7 is localized to 13q12 locus [GenBank Accession U69566]. However, two more tumor suppressor genes have been found in this region of the chromosome: breast cancer susceptibility (BRCA2) gene was localized to chromosome 13q12 [Wooster, R. et al., Science, 265: 2088-2099 (1994)], and retinoblastoma (RB1) gene was localized to 13q14 [Walbaum, R., et al., Hum. Genet., 44: 219-226 (1978)].

As the rabbit HCD10(119) was inhibited by human growth hormone according to the present inventors' experiments, another approach to uncover the types of cancers affected by hGHITS gene is to make a survey of any correlation between the elevation of the amount of growth hormone or insulin-like growth factor-1 (IGF-1) and the development of respective cancers.

For example, receptors for IGF-1 have been found in several different types of tumors including prostatic cancer [Lamharzi, N. et al., Proc. Natl. Acad. Sci. USA, 95: 8864-8868 (1998)], pancreatic cancer [Bergmann, U., et al., Cancer Res., 55: 2007-2011 (1995)], bone and soft-tissue sarcoma [Sekyi-Otu, A. et al., Cancer Res., 55:129-134 (1995)], Wilms' tumor, small cell lung carcinoma, colonic cancer, meningioma, neuroblastoma, rhabdomyosarcoma and lymphoblastic leukemia [Daughaday, W.H., Endocrinology, 127: 1-4 (1990)]. Some tumors are known to be activated by growth hormone or IGF-1. For example, acute leukemia is known to be activated by human growth hormone to upregulate its receptors [Giesbert, S. et al., Ann. Hematol., 74: 253-257 (1997)]

As is expected from the above, use of growth factor antagonists will result in inhibition of some tumors: the growth of human osteosarcoma can be inhibited by a somatostatin analog [Pinski, J. et al., Int. J. Cancer, 65: 870-874 (1996)]. A growth hormone-releasing hormone antagonist inhibits the proliferation of human renal adenocarcinoma [Jungwirth, A. et al., Proc. Natl. Acad. Sci. USA, 94: 5810-5813 (1997)].

There was found a positive correlation between patients' height and their incidence of developing some tumors. For example, a study suggests that medulloblastoma may be influenced by growth hormone production [Robertson, S.C. et al., Neurosurgery, 41: 561-565 (1997)]. However, there is an unresolved contradiction in the case of breast cancer. Some researchers believe that IGF-1 is a potent breast mitogen [Yang, X.F. et al., Cancer Res., 56: 1509-1511 (1996)]. They implicated growth hormone in human mammary growth, and showed that gynecomastia occurred in some children treated with growth hormone, and that tall stature and acromegaly were associated with an increased incidence of breast cancer [Ng, S.T. et al., Nat. Med., 3: 1141-1144 (1997)]. However, other researchers believe that they have found that the age when a woman reaches her maximum height (not the total attained height) is a risk factor for breast cancer [Li, C.I. et al., Epidemiology, 8: 559-565 (1997)]. There is allegedly a trend of decreasing risk of breast cancer in relation to increased age of final height attainment.

Findings on patients with acromegaly contributed exceptional information pertaining to diseases induced by high level of growth hormone. The boosted level of growth hormone in acromegaly patients results in increased risk of developing colonic adenomas [Vasen, H.F. et al., Eur. J. Endocrinol., 131(3): 235-237 (1994)], colorectal cancer [Jenkins, P.J. et al., Clin. Endocrinol. Oxf., 47(1): 17-22 (1997)], cardiovascular diseases [Lombardi, G. et al., J. Pediatr. Endocrinol. Metab., 10: 553-560 (1997)], and all malignant disease [Orme, S.M. et al., J. Clin. Endocrinol. Metab., 83: 2730-2734 (1998)].

Reduction of proliferative activity of tumor cells and improvement of cardiac function during octreotide (analog of somatostatin) treatment with simultaneous lowering of serum IGF-1 level confirm that growth hormone is a cause of above diseases [Cascinu, S. et al., Gastroenterology, 113: 767-772 (1997), Lombardi, G. et al., Horm. Res., 48 Supple. 4: 38-42 (1997) ]. In the case of cardiovascular diseases it seems that the root of the illness is not just growth of ventricular mass because it is disproportionate to the increase in size of other internal body organs, and the severity of the acromegalic cardiomyopathy was reported to be correlated better with the "disease duration" than with circulating growth hormone and/or IGF-1 levels [Lombardi, G. et al., J. Endocrinol., 155 Supple. 1: S33-S37 (1997)]. That makes us surmise an existence of gene(s) suppressed by some threshold levels of growth hormone resulting in development of cardiovascular diseases.

Moreover, with colon as a prime target, Northern hybridization was carried out using an equal amount of mRNA extracted from various normal and cancer cells and with hGHITS-specific probes. At least ten times increase of hGHITS transcription was observed in colonic adenocarcinoma SW480 compared with normal colonic tissue (data not shown). The expression level was obviously the highest among eight tested cultures of cancer cells. This suggests that some dramatic mutation(s) in the colonic adenocarcinoma has results in nonfunctional homologue of the gene.

While increased transcription of functional hGHITS is expected to act suppressively on tumor growth, absolutely unexpected result was obtained for the cell culture of Burkitt's lymphoma Raji, which lacked any visible expression of hGHITS mRNA (data not shown). This result suggests the possibility that a defect of hGHITS gene has caused the development and proliferation of tumor in Burkitt's lymphoma.

The above findings as a whole indicate the possibility that the DNAs, their transcription product mRNAs and the translated proteins of the present invention can be utilized as therapeutic drugs by introducing the DNAs composing the gene of the present invention into the patients or by applying the mRNAs or the proteins to the affected sites, for treatment of cardiopathy, angiopathy, diabetic nephropathy and malignant tumors such as breast cancer, colorectal cancer and leukemia, which could be induced by elevated levels of growth hormone or IGF-1 in such situations where excessive secretion or dosage of growth hormone occurs as in gigantism/acromegaly or in dwarfism. In addition, the present invention also suggests the possibility that certain substances that serve to induce or promote the expression of the gene of the present invention, e.g., such substances that stimulates transcription of the gene of the present invention as concanavalin A, can be utilized as therapeutic drugs for treatment of the above diseases.

In addition, by utilizing the correlation between hGHITS gene expression and cancer development, DNAs comprising part (or the whole) of the DNAs composing hGHITS gene could provide means for cancer diagnosis based on the status of hGHITS gene expression as well as means for prognosis in cancer patients, through provision of, for example, probes or PCR primers for the transcript of the hGHITS gene.

According to etiological studies, the incidence of such diseases as tumor development and angiopathy is greater in patients suffering from gigantism or acromegaly, who are under sustained excessive levels of growth hormone. This suggests that these diseases, i.e. tumor development and angiopathy, may result from the inhibition of the tumor suppressor gene of the present invention caused by sustained excessive levels of growth hormone in the blood. In addition, physiological secretion of growth hormone occurs in a pulsatory manner. Thus, "duration" of growth hormone levels above a threshold value is expected to be responsible for the inhibition of the expression of the gene of the present invention. This suggests that, also in a growth hormone therapy, not a method effecting continuous administration but a method that enables a pulsatory pattern of administration is preferable in view of safety which is in conformity with a natural, physiological cycle. In general, it is also desirable to refer to the genetic information of individual patients in selecting drugs and determining their doses for treating the patients.

In treating a child with dwarfism with growth hormone, therefore, probes and primers designed upon the gene and their analogue of the present invention may be used as diagnostic drugs for measuring the levels of expression of the tumor suppressor gene of the present invention, and the results thus obtained then may be utilized for assessing the adaptability of the patient to the treatment and for determining proper term and doses for treatment, thus lowering the risk of tumor development.

Although a large-scale research is required to reach the final conclusion, it is reasonable to expect that in the human population there is a positive correlation between the low hGHITS gene expression levels or expression of their nonfunctional alleles and the risk of future development of cancers. Therefore, by examining an individual for expression of hGHITS gene using probes and primers obtained based on the gene of the present invention, it will become possible to give a preliminary diagnosis on the probability of future tumor development in the individual. In addition, besides such a monitoring, also suggested is a possibility that proper substances selected from those that can promote or induce the expression of hGHITS gene, such as concanavalin A, can be utilized to prevent the development of a tumor.

The DNAs of the present invention include not only the DNA having the nucleotide sequences specifically set forth under SEQ ID NO:1 or SEQ ID NO:2 in the Sequence Listing, but also a DNA composing a gene substantially of the same property and having deletion, substitution or addition of one or more bases relative to either of those nucleotide sequences. The term "one or more bases" generally includes to up to 10 bases, and usually up to a few (e.g., 3 or 4) bases. The proteins of the present invention include not only the proteins having the nucleotide sequences specifically set forth under SEQ ID NO:3 and SEQ ID NO:4, but also a protein substantially of the same property and having deletion, substitution or addition of one or more amino acids relative to either of those amino acid sequences. The term "one or more amino acids" generally includes up to 10 amino acids, and usually up to a few (e.g., 3 or 4) amino acids. Furthermore, the present invention includes DNA sequences coding such proteins. A variety of such nucleotide sequences can be readily prepared making use of the familiar knowledge on degeneracy of the codon.

Using recombinant DNA technology, a variety of mutants is readily available. First, it is possible to introduce a mutation into a DNA clone fragment through different chemical and/or enzymatic processes, and the mutants DNA thus obtained are then sequenced to select particular mutants with an intended merit. This method allows a systematic preparation of different mutants regardless of their phenotypes. General methods of preparing a mutant clone DNA are as follows:
1. With the help of an oligonucleotide, substitution, deletion, insertion or addition can be directly carried out in a given DNA sequence. This method enables to introduce a number of mutations in a small region of a DNA.
2. Using a longer oligonucleotide, it is possible to synthesize a desired gene.
3. By means of region-specific mutagenesis, a desired mutation can be introduced into a large (1 - 3 kb) DNA region.
4. Linker-scanning mutagenesis of DNA is a method suited for introducing a cluster point mutation into a relatively small (4 - 10 bp) DNA region
5. PCR is also utilized as a method for direct introduction of a mutation. [References: Current Protocols in Molecular Biology., 3 Vols., Edited by Ausubel F.M. et al., John Wiley & Sons, Inc., Current Protocols., Vol. 1, Chapter 8: Mutagenesis of Cloned DNA, pages 8.0.1-8.5.10]

Methods of preparing plasmids and vectors which can express a desired gene including a different mutation obtained by the above methods. That is, by inserting a DNA carrying a desired gene into the DNA of a expression vector using a combination of restriction enzymes and a ligase, a recombinant plasmid is readily constructed carrying the desired gene. The recombinant plasmid thus obtained is then introduced into different cells to transfect them, thereby producing transformed cells. Cells ranging from prokaryotes, e.g. E. coli, to yeast, insects, plants and animal cells may be utilized. [References: Vectors Essential Data. Gacesa P. and Ramji D.P., 166 pages. BIOS Scientific Publishers Limited 1994., John Wiley & Sons in association with BIOS Scientific Publisher Ltd. Expression vectors, pages 9-12.]

Introduction of a recombinant plasmid into host cells is effected by calcium chloride method or electroporation. Calcium chloride method provides efficient transformation and requires no special apparatus. For higher efficiency, electroporation is recommended.
[References: Current Protocols in Molecular Biology, 3 Vols. Editied by Ausbel F.M. et al., John Wiley & Sons, Inc., Current Protocols, Vol. 1, unit 1.8: Introduction of Plasmid DNA into Cells, pages 1.8.1-1.8.10]

Two types are known of transfection generally carried out on animal cell lines, i.e. transient and permanent types. In transient transfection, transformed cells are cultured for 1 - 4 days to effect transcription and replication of the transfected gene, and then the cells are harvested and their DNA analyzed. Alternatively, in many studies, a stable transformant cell line is produced, in which the transfected gene is incorporated into the chromosomes. Examples of the method for transfection include calcium phosphate method, electroporation, and liposome fusion method.
[Reference: Current Protocols in Molecular Biology, 3 Vols. Edited by Ausubel F.M. et al., John Wiley & Sons, Inc., Current Protocols. vol. 1, Chapter 9: Introduction of DNA into Mammalian Cells, pages 9.0.1-9.17.3.]

Preparation of polyclonal and monoclonal antibodies directed against the proteins (polypeptides) coded by the tumor suppressor gene of the present invention, or against their fragments and analogues as well, are readily carried out using techniques well known in the art. When purified, the antibodies may be used as laboratory reagents and diagnostic agents for diseases associated with the tumor suppressor gene of the present invention. The antibodies obtained are used for preparation of antibody columns, immunoprecipitation as well as for identification of the antigen by Western blotting.

A general method for preparing a monoclonal antibody in mg-scale against the proteins coded for by the tumor suppressor gene of the present invention is as follows: Mice are inoculated with the antigen protein to immunize, and the spleen is removed from the mice exhibiting a sufficient antibody titer. The spleen cells are separated, and B cells selected are fused with myeloma cells of B cell origin to form hybridoma cells which secrete the antibody. The monoclonal antibody secreted from the hybridoma cells is purified from the culture medium by means of an affinity column, ion-exchange, or gel filtration, etc. The polyclonal antibody of the present invention also may be prepared by a conventional method: Using rabbits, horses, mice or guinea pigs as immunized animals, the antigen protein is inoculated along one of the schedules known in the art to immunize the animals, and then IgG, etc. are isolated from the collected serum.
[Reference: Current Protocols in Molecular Biology, 3 Vols. Edited by Ausubel F.M. et al., John Wiley & Sons, Inc., Current Protocols, Vol. 2, Chapter 11: Immunology, pages 11.0.1-11.16.13.]

### EXAMPLE

The present invention is described in further detail with reference to the example below. However, it is not intended that the scope of the present invention be restricted to the example.

### <Preparation of Primers>

Primers specific to hGHITS were designed using DNASIS software and OLIGO software. M13 universal sequencing primer (USP) (gtaaaacgacggccagt: SEQ ID NO:5) and M13 reverse sequencing primer (caggaaacagctatgac: SEQ ID NO:6) for sequencing of insertions in pUC18 vector, and following hGHITS-specific primers were ordered to Nisshinbo Tokyo Research Center:
H119U89 (tccaaatattaccagaaagggagcca: SEQ ID NO:7),
H119U142 (gacctctgggatgatgtgt: SEQ ID NO:8),
H119U219 (agaggtcttgggcacatttcactggt: SEQ ID NO:9),
H119U736 (cctgagcacgcattttacg: SEQ ID NO:10),
H119U782 (acaatggctacccctttcg: SEQ ID NO:11),
H119L739 (ttcgtaaaatgcgtgctc: SEQ ID NO:12),
H119L1048 (cctgtttgggttttcttggt: SEQ ID NO:13),
H119L1493 (tcatcaccttgctcacacttccctatta: SEQ ID NO:14),
H119L1499 (catcaccttgctcacacttcc: SEQ ID NO:15),
H119L1515 (gtcggaaatcacagccacatcatca: SEQ ID NO:16),
H311U13 (ttcgtttgcgtcctaccac: SEQ ID NO:17),
H311L320 (gcggcgtcttgctctg: SEQ ID NO:18),
H316U39 (ctgctctggtctcaatttaag: SEQ ID NO:19),
H316L498 (caagtctgctgtgcctgtc: SEQ ID NO:20).

Forward T7 promoter primer (ctaatacgactcactataggga: SEQ ID NO:21) and reverse U-20mer primer (ggttttcccagtcacgacgt: SEQ ID NO:22) for sequencing of insertions in cloning pMOSBlue T-vector were synthesized using a DNA synthesizer, Model 391 PCR-mate EP™ (Applied Biosystems, USA).

### <Search for a Suitable Human cDNA Library>

QUICK-Screen Human cDNA Library Panel (Clontech) containing aliquots of 14 separate cDNA libraries and an aliquot of combined libraries (Universal cDNA Library) were used in the search for a library with the best representation of hGHITS gene.

10 µl each of reaction mixtures was prepared in MicroAmp Reaction Tubes With Caps (Perkin Elmer): 6.4 µl of H₂O, 1 µl of 10 × amplification buffer (100 mM Tris-HCl, pH 8.3, 500 mM KCl, 15 mM MgCl₂, 0.01 % w/v gelatin), 0.2 µl of dNTP mix (2.5 mM each), 1 µl of the forward primer (2 µM), 1 µl of the reverse primer (2 µM), 0.2 µI of a heat-treated cDNA library aliquot, and 0.2 µl of a polymerase mixture [0.1 µl of AmpliTaq DNA Polymerase (5 U/µl) (purchased from Perkin Elmer) and 0.1 µl of TaqStart Antibody (7 µM, 1.1 µg/µl) (purchased from Clontech)]. The blends were pipetted up and down to mix well. PCRs were carried out in a GeneAmp PCR System 9600 thermal cycler (Perkin Elmer) according to the following amplification cycles:
[40 cycles repeated]
10 sec: 96 °C (denaturation)
1 min: 56 °C (annealing)
1 min: 72 °C (extension)
[Final step] 4 °C (hold)

Fractionation gel was prepared by melting 1 % Agarose GP-36 (Nacalai Tesque) in 0.5 x TBE buffer (45 mM Tris, 45 mM boric acid, 1 mM EDTA, pH 8.3) containing 0.5 µg/ml ethidium bromide with microwave oven, pouring the solution into a 110 x 60 x 7.5 mm mould of Mupid-2 Mini-Gel Electrophoresis System (Advance), and allowing to solidify at room temperature for at least 30 min with an inserted comb.

Each PCR reaction mixture was mixed with 2 µl of 10 x Loading buffer (50 % glycerol, 0.01 % bromphenol blue) and applied to the gel. Gel electrophoresis was run at 100 V for 30 min using Mupid-2 Mini-Gel Electrophoresis System containing 0.5 x TBE buffer in the buffer chambers.

The pictures were taken while transilluminating the gel with a transilluminator. Proper PCR fragments were excised from the gel.

### <Purification of PCR Fragments>

QIAEX II Gel Extraction Kit (QIAGEN) was employed for the purification of DNA fragments. Three volumes of Buffer QX1 were added to one volume of the gel for DNA fragments of 100 bp-4 kbp. QIAEX II was resuspended by vortexing for 30 sec, and 10 µl of the resin was added to each of the samples. The mixtures were incubated at 50 °C for 12 min in a Thermo Alumi Bath ALB-120 (Iwaki). The samples were briefly vortexed every 2 min. The tubes were centrifuged at 18,000 x g (15,000 rpm on TOMY MRX-150 High Speed Micro Refrigerated Centrifuge) for 1 min and the supernates were discarded. 500 µl of Buffer QX1 was added to each DNA containing pellet, briefly vortexed, centrifuged at 18,000 x g for 1 min and supernates was completely removed. 500 µl of Buffer PE was added to each DNA containing pellet, briefly vortexed, centrifuged at 18,000 x g for 1 min and the supernates was completely removed. This washing step was repeated one more time. The pellets were air-dried for 25 - 30 min until they turn white. Vacuum drying was evaded because overdried pellets would lead to lower recoveries. Purified water was added to each sample, the pellets were resuspended by vortexing and incubated at room temperature for 5 min occasionally vortexing the mixtures. The sample were centrifuged at 18,000 x g for 1 min and the DNA containing supernates were transferred to clean tubes. This step was repeated with incubating the mixture at 50 °C. First and second DNA eluates of the same PCR product were combined, and 1 µl aliquot was tested by agarose electrophoresis. Purified DNA fragments were stored at -20 °C.

### <Cloning of the Purified PCR Fragments>

A pMOSBlue blunt ended cloning kit (Amersham) was used for cloning of the thus amplified and purified band products. The 3.75 µl of each purified concentrate was mixed in a 1.5-ml tube with 0.5 µl of 10 × pk buffer, 0.25 µl of 100 mM DTT and 0.5 µl of pk enzyme mix. The mixtures were stirred gently with pipette tips and briefly spun in a microcentrifuge. The tubes were incubated at 22 °C for 40 min in a Sanyo Incubator. The reactions were heat inactivated at 75 °C for 10 min in the Thermo Alumi Bath, cooled on ice for 2 min and centrifuged briefly to collect the condensate.

Each ligation reaction was prepared by mixing 5 µl of the above product with 0.5 µl of pMOSBlue vector (50 ng/µl) and 0.5 µl of T4 DNA ligase (2 Weiss units). The mixtures were stirred gently with pipette tips and briefly spun in a microcentrifuge. The tubes were incubated overnight at 22 °C in a Sanyo Incubator.

One tube containing 200 µl of pMOSBlue competent cells was thawed and stirred to evenly mix the suspend cells. The 15 µl of the competent cells was pipetted into each of the prechilled 1.5-ml tubes. 1 µl of the ligation mixture was added directly to the cells and stirred gently to mix. The remaining cells was frozen again and used for next transformations (with some loss of transformation efficiency). The tubes were left on ice for 30 min. The cells were heat-shocked for exactly 45 sec at 42 °C in the Thermo Alumi Bath ALB-120 and placed on ice for 30 sec. The 80 µl of SOC medium at room temperature was added to each of the tubes, and the tubes were left at 37 °C for 1 hr.

Plates containing 1.5 % Bacto-Agar (Difco), 30 capsules/L of CircleGrow (Bio 101, Inc.), 75 µg/ml ampicillin and 15 µg/ml tetracycline were spread with 100 µl of 20 mg/ml X-Gal (5-bromo-4-chloro-3-indolyl-β-D-galactoside) and 20 µl of 100 mM IPTG (isopropyl-β-D-thio-galactopyranoside). The agar plates were left to soak for at least 30 min before plating.

Total volume of each transformed cell suspension was spread onto the agar plates. Inverted plates were incubated overnight at 37 °C. Four different phenotypes were observed: dark blue, light blue, white with a blue center (so-called "bull's eye") and white. Insertions were found only within the white plaques.

### <Purification of Plasmid DNAs>

White colonies containing the transformants were picked up from the plate using sterile toothpicks. The bacteria were transferred to 14-ml polypropylene tubes with caps (Falcon) containing 2.5 ml of CircleGrow medium (40 capsules/L) with 100 µg/ml ampicillin. The tubes were shaken at 200 rpm at 37 °C for 16 - 72 hrs (1 day cultivation gives the maximal yield of plasmid DNA).

RPM AFS Kit (Bio 101, Inc.) was employed for rapid isolation and purification of double-stranded DNAs from the bacterial cultures. Up to 12 plasmid DNAs were purified at once. Bacterial cells were spun in 2-ml Safe-Lock Tubes (Eppendorf) at 18,000 x g (15,000 rpm on TOMY MRX-150 High Speed Micro Refrigerated Centrifuge) for 1 min. Supernates were decanted and discarded. The cells were resuspended in 1 ml water by vortexing and spun again. Supernates were decanted and discarded. Each cell pellet was resuspended in 200 µl of Pre-Lysis Buffer #1 by vortexing until the cells were completely resuspended. 400 µl of Alkaline Lysis Solution #2 was added directly into the cell suspensions, and the tubes were gently inverted 15 times. One min later, 300 µl of ice-cold Neutralizing Solution #3 were added, and the tubes were shaken vigorously 3 - 5 times until a uniform white precipitate formed. The mixtures were incubated for 5 min on ice, spun 5 min at room temperature, and the supernates were transferred to new 2 ml tubes.

The 500 µl of Glassmilk SpinBuffer #4 was added to the supernates, turned up and down for 5 min for efficient binding of the DNA to the Glassmilk, and spun at 18,000 × g for 2 sec. The supernates were decanted and discarded. Each Glassmilk/DNA complex was resuspended in 500 µl of Wash Solution #5 by gently stirring with a pipette tip followed by pipetting up and down, and transferred to a kit-supplied RPM AFS Spin Filter. The filter was spun for 10 sec to lower the level of the wash solution to the level of the pellet without drying it. The Catch Tube was emptied, and the Spin Filter was reassembled. The 500 µl of Wash Solutions were added to each Spin Filter followed by centrifugation for 2 min to dry filter content. The spin filters were transferred to new kit-supplied RPM AFS Catch Tubes. The 140 µl of Elution Solution #8 (RNase/DNase/pyrogen free H₂O) was added to each sample, and the Glassmilk/DNA complex was mixed to a slurry by gentle finger tapping. The plasmid DNAs were collected in the Catch Tubes by centrifugation at 15,000 rpm for 3 min. The DNA solutions were stored at -20 °C.

### <Detection of DNA Inserts>

The presence of insertions and appropriate amount of DNA solution for DNA sequencing were determined by agarose electrophoresis of plasmid DNAs digested with restriction endonucleases. Two µl each of plasmid DNA solutions was mixed with 11.1 µl of H₂O, 1.5 µl of 10 × H Universal Buffer (0.5 M Tris-HCl, pH 7.5, 100 mM MgCl₂, 10 mM DTT, 1 M NaCl), 0.2 µl of Sail (10 U/µI) (purchased from Takara), 0.2 µl of EcoRI (10 U/µI) (purchased from Takara), and incubated at 37°C for at least 2 hrs.

Separation gel was prepared by melting, in a microwave oven, the mixture containing 1 % Agarose GP-36 (Nacalai Tesque), 0.5 µg/ml Ethidium Bromide (Sigma) and 0.5 x TBE buffer, pouring the solution into the mould of Mupid-2 Mini-Gel Electrophoresis System (Advance), and allowing to solidify at room temperature for at least 30 min.

Each of the digested plasmid DNA was mixed with 1.5 µl 1 of 10 x Loading buffer (50 % glycerol, 0.01 % bromphenol blue) and applied to the gel. pHY marker (Takara) was used as a DNA molecular weight standard marker. Gel electrophoreses were run at 100 V for 30 min using Mupid-2 Mini-Gel Electrophoresis System containing 0.5 × TBE buffer in the buffer chambers. The pictures were produced using FAS-II (Toyobo): the gels were illuminated with an electronic U.V. transilluminator, the pictures were taken with XC-75/75CE CCD Video Camera Module (Sony) and printed with Video Graphic Printer UP-880 (Sony). DNAs from clones carrying insertions with proper lengths were chosen for DNA sequencing to get the final confirmation.

### <Preparation of Samples for DNA Sequencing>

ABI PRISM™ Dye Terminator Cycle Sequencing Ready Reaction Kit with AmpliTaq DNA Polymerase, FS was employed for DNA sequencing. Up to 18 PCR products were processed at one time. The reaction mixtures were prepared in MicroAmp Reaction Tubes With Caps (Perkin Elmer) as follows: 8 µl of Terminator Ready Reaction Mix, 2 µl of forward or reverse primer (2 µM, 0.1 - 0.5 µg of plasmid DNA (the required volume of DNA solution was estimated based on the electrophoretic pictures) and H₂O up to a final volume of 20 µl. The blends were pipetted up and down to mix well. PCRs were carried out in the GeneAmp PCR System 9600 thermal cycler (Perkin Elmer) using the following amplification cycles:
[25 Cycles Repeated]
10 sec: 96 °C (denaturation)
5 sec : 50 °C (annealing)
4 min: 60 °C (extension)
[Final step] 4 °C (hold)

PCR products were purified with ethanol precipitation. For each reaction product, a 1.5-ml microcentrifuge tube was prepared by addition of 2 µl of 3 M sodium acetate (pH 5.2) and 50 µl 1 of 99.5 % ethanol. The entire 20-µl 1 content of each reaction tube was transferred to the microcentrifuge tube containing the ethanol solution. The tubes were vortexed and placed on ice for 10 min. The mixtures then were centrifuged at 18,000 x g (15,000 rpm on TOMY MC-150 High Speed Micro Centrifuge) for 20 min at 4 °C. The ethanol solution was carefully aspirated with a micropipette. The pellets were rinsed by addition of 250 µl of 70 % ethanol and centrifuged at 15,000 rpm for 1 min. The alcohol solution was aspirated with a micropipette carefully to avoid disturbing the pellets. The tubes were dried under vacuum for 10 min. The dried precipitates were used immediately or stored at -20 °C.

### <DNA Sequencing>

The 373-18 DNA Sequencer (Applied Biosystems) was employed for sequencing the plasmid DNAs. Since the cleanness of glassware is highly important for credible, accurate sequencing, the glassware was carefully washed with water and then wiped with isopropyl alcohol. The 4.75 % denaturing PAAG with 8.3 M urea was used for separation of PCR products: 19.94 g of urea were mixed in a 50-ml centrifuge tube with 4.75 ml of 40 % (19:1) Acrylamide/Bis Solution (Bio-Rad Laboratories) and 8 ml of 5 x TBE Buffer, and distilled water was added to make 40 ml. The tube was vigorously stirred, warmed during 1 turn of the dish in a microwave stove and vigorously stirred again until all urea crystals dissolved. The solution was degassed in a desiccator equipped with am A-3S aspirator while the gel casting equipment was prepared (approximately 10 min). 18 µl of TEMED (Sigma) and 160 µl of 10 % ammonium persulfate (stored at -20°C) were added, gently mixed for 30 sec and poured into a glass plate mould (420 x 250 x 0.25 mm). The gel was allowed to solidify at room temperature for 2 - 5 hrs.

A loading buffer was prepared by mixing 52 µl 1 of formamide with 10 µl 1 of 3 % blue dextran (Sigma) in 50 mM EDTA (pH 8.0). The sequencing samples were dissolved in 3 µl of this buffer, heated at 94°C for 2 min in the Thermo Alumi Bath ALB-120 (Iwaki) and then kept on ice. The glass plates with the gel were carefully washed once more, checked by scanning, and the electrophoresis chamber was assembled. The shark teeth comb was inserted at 1 mm dip, 1 × TBE buffer was poured into the both buffer chambers, and pre-electrophoresis performed for 20 - 30 min. The PMT voltage was set at 900 V, run parameters were set at 2500 V, 20 mA and 30 W. The wells were carefully washed with 1 × TBE buffer using a syringe, the samples loaded in the wells, and electrophoresis was carried out for 9 hrs. The Data Collection computer program was run immediately after the electrophoresis was started.

ANALYSIS computer program was activated automatically after the completion of data collection. Software's mistakes were corrected manually. The initial analysis of the nucleotide sequences was performed using DNASIS software.

### <Preparation of Hybridization Probes>

Hybridization probes were prepared from the stock solutions of plasmid DNAs with suitable insertions as described under "Detection of insertions". Purified DNA samples were mixed with 0.116 volume of 10 x H Universal Buffer, and 0.02 volume each of SalI (10 U/µl) and EcoRI (10 U/µl). Digestion proceeded overnight at 37°C. Agarose gel electrophoresis was run at 50 V for 50 min, and the DNA insertions were excised from the gel, identical ones combined and purified as described under "Purification of PCR Fragments".

### <Preparation of Phage Plating Cells>

To obtain a high efficiency of phage infection, it is important that the cells used for phage growth are harvested in the logarithmic growth phase and are substantially free of dead cells. The XL1-Blue MRF' strain was found to be the most convenient to use. Its genotype is: Δ(mcrA)183 Δ (mcrCB-hsdSMR-mrr)173 endA1 supE44 thi-1 recA1 gyrA96 relA1 lac[F'proAB lacl^{q}ZΔM15 Tn10(Tet^{r}]. The strain was cultured on CG agar plate (1.6 % Bacto-Agar (Difco) and 40 Capsules/L of CircleGrow (Bio 101, Inc.)) supplemented with 25 µg/ml tetracycline. The cells were restreaked onto a fresh plate every fortnight, incubated overnight at 37°C and stored at 4°C.

The day before the plating stage, a single colony was picked up from the CG agar plate, inoculated into 3 ml of CG broth (40 Capsules/L of CircleGrow) supplemented with 0.4 % maltose and 25 µg/ml tetracycline, and incubated overnight with shaking at 37°C.

1.0 ml of the overnight culture was added to 47 ml of the same pre-warmed growth medium and incubated for 3 hrs at 37°C with vigorous shaking. The cell concentration was estimated using a Beckman DU 640 spectrophotometer equipped with 0.1-ml quartz glass cuvettes. The OD₆₀₀ was usually around 0.3 (1.5 × 10⁸ cells/ml). The culture was transferred to a sterile 50-ml polycarbonate centrifuge bottle with a cap (Beckman) and spun at 3,000 rpm for 10 min at 4°C in a Beckman J2-MC Centrifuge using a Beckman JA 20 rotor. The cell pellet was resuspended in ice-cold sterile 10 mM MgSO₄ to prepare 2 × 10⁹ cells/ml suspension. The cells were placed on ice and immediately used for titration and plating of phages for screening.

### <Titration of Phages>

One hundred-mm Petri dishes (Falcon) containing CG agar supplemented with 25 µg/ml tetracycline were used for titration. The dishes in which the agar was poured were allowed to stand with their lids open at room temperature for 20 min to solidify the agar, then dried for 30 min by blowing sterile air in a safety cabinet (SCV-1303EC II B: Hitachi) and stored at 4°C. Before use, they were taken out of the refrigerator and warmed at 37°C for 1 hr in a constant-temperature room.

Two µl of phage mixture were mixed with 198 µl of SM buffer (100 mM NaCl, 8 mM MgSO₄, 0.01 % gelatin (Sigma) and 50 mM Tris-HCl, pH 7.5). This dilution was labeled 10⁴ since, when 10 µl of the dilution was plated, one plaque on the plate was equivalent to 10⁴ pfu/ml in the original phage stock. The successive dilutions down to 10⁹ were prepared in the same manner with SM buffer .

Ten µl of each phage dilution was diluted with 90 µl of SM buffer, mixed with 100 µl of cells suspended in 10 mM MgCl₂ and incubated in a metal holder of the Thermo Alumi Bath ALB-120 at 37°C for 15 min.

CG top agar (0.8 % Type I-B Agarose (Sigma), 40 Capsules/L of CircleGrow) was melted in a microwave stove and supplemented with 20 % maltose up to 0.4 % final concentration. The 3-ml aliquots of top agarose were added to 16 × 125-mm tissue culture tubes with screw caps (Falcon) and kept before use at 48°C in the Thermo Alumi Bath ALB-120.

Each plating compound was added to the tube containing the melted agarose, mixed quickly by inverting the tube 5 times, and poured onto the dry CG agar in 100-mm plates supplemented with 25 µg/ml tetracycline. The top agarose was allowed to set completely for 3 min before moving the plates. The plates were inverted and incubated at 37°C for 5 hrs in a constant-tempreture room, then overnight at room temperature. The number of plaques was counted and the phage titer was calculated by multiplying the total number of plaques by the dilution number.

### <Plating of Phages and Transfer of Plaques onto Membranes>

The plating protocol is basically the same as described above about titration of phages. Up to 12 plates were processed at one time. The cDNA library was constructed with λgt10 cloning vector by oligo(dT) + random priming method using a lung from a 75-year-old Caucasian male who had died because of trauma (Clontech).

For first screening, 100 µl of the phage library diluted with SM buffer (containing 50,000 - 500,000 pfu (plaque forming units)) were mixed with an equal volume of cells suspended in 10 mM MgCl₂, incubated for 15 min at 37°C, mixed well with 7 ml of CG top agar at 48°C by 5 inversion, and poured onto a CG agar plate (150 mm: Falcon) supplemented with 25 µl/ml tetracycline. The plates were inverted and incubated at 37°C for 5 hrs, and then overnight at room temperature.

Hybond™-N+ (round, 132-mm, gridded, positively charged nylon membrane (Amersham)) was placed on a plaque-containing agar plate. The orientation of the filter was marked at three points with a 21G needle. The filter was removed and placed with its face up on the table and dried for at least 3 min. For DNA fixation, the filter was placed on 2 sheets of Whatman paper saturated with 0.4 M NaOH for 10 - 30 min. To remove bacterial debris and alkali, the membrane was thoroughly washed twice with 5 x SSPE buffer (20 × SSPE: 3.0 M NaCl, 0.2 M NaH₂PO₄, 0.02 M EDTA, pH 7.4, from GibcoBRL) and once with water, blotted with a paper towel and dried at room temperature.

### <Labeling and Purification of Probes>

Multiprime DNA labeling system (Amersham) were used for labeling probes. Approximately 25 ng of each recovered DNA insert was adjusted to 27 µl with water, heated at 98°C in the Thermo Alumi Bath ALB-120 for 5 min and chilled on ice. The samples were centrifuged at 18,000 × g (15,000 rpm on TOMY MRX-150 High Speed Micro Refrigerated Centrifuge) for 1 sec to pull down the tube contents. Each of the four reactions was set up on ice in MicroAmp Reaction Tube With Cap as follows: the total volume of the denatured DNA probe, 10 µl of Labeling buffer, 5 µl 1 of Primer/BSA solution, 6 µl of [α-³²P]dCTP (~110 TBq/mmol, 370 MBq/ml) (Amersham) and 2 µl of Klenow enzyme. The blends were mixed gently by pipetting up and down, and incubated in a GeneAmp PCR System 2400 at 37°C for 30 min, kept at 20°C for 3 - 4 hrs until removal of unincorporated nucleotides and then hybridization.

QIAquick Nucleotide Removal Kit (Qiagen) was used for purification of the labeled probes. Each labeling mixture was mixed with 500 µl of Buffer PN in a 1.5-ml tube. A QIAquick spin column was placed in a provided 2-ml collection tube. To bind the DNA, the sample was applied to the QIAquick spin column and centrifuged for 1 min at 6,000 rpm (TOMY High Speed Micro Centrifuge MC-150). The QIAquich column was placed into a clean 2-ml collection tube, and the radioactive flow-through was retained for subsequent calculation of the label incorporation into the DNA. To wash QLAquick column, 500 µl of Buffer PE was added and centrifuged at 6,000 rpm for 1 min. The flow-through was discarded and washing repeated with another 500µl of Buffer PE. The flow-through was discarded, and the QIAquick column was centrifuged for additional 1 min at 13,000 rpm to remove the remaining fluid. The QIAquick column was placed in a clean 1.5-ml microcentrifuge tube. To elute the DNA from the column, 100 µl of Buffer EB (10 mM Tris-HCl, pH 8.5) was added to the center of the column, let stand for 1 min, and then centrifuged for 1 min at 13,000 rpm.

Each of the labeled DNAs eluted was transferred into a MicroAmp Reaction Tube With Cap, heated in a GeneAmp PCR System 2400 for 5 min at 99.9°C and then chilled to 4°C. The denatured probe was added to a 1.5-ml tube containing 450 µl of hybridization buffer and the efficiency of label incorporation was evaluated using β (γ) Survey Meter TGS-133 (Aloka) and compared with the radioactivity of unincorporated nucleotide.

### <Plaque Hybridization>

Hybridization and filter washing were performed at low temperatures to be beneficial for precise positioning of filters to excise positive plaques. The 100 ml of hybridization buffer was prepared as follows: 50 ml of Formamide, deionized, Nuclease and Protease tested (Nacalai Tesque), 25 ml of 20 × SSPE Buffer (3M NaCl, 0.2 M NaH₂PO₄, 20 mM EDTA, pH 7.4: GibcoBRL), 5 ml of 10 % SDS, 18 ml of water; two 1.5-ml tubes each containing 200 µl of 10 mg/ml Herring Sperm DNA Solution (GibcoBRL) and 0.8 ml of water were heated at 98°C in the Thermo Alumi Bath ALB-120 for 5 min, chilled on ice, and merged with the hybridization buffer. As SDS was prone to precipitate with decreasing temperature, the hybridization buffer was warmed (in winter season) to about 40°C just before use.

The 80 ml of the hybridization buffer was poured into a 150-mm round plastic container and 12 filters were immersed one by one taking care so that the front face of each membrane touches with the front face of the adjacent membrane and the back face of each membrane with the back face of the adjacent membrane. The lid was closed and the prehybridization was performed in a Thomastat Shaker T-22S at 32°C, at 40 rpm for at least 3 hrs.

The hybridization buffer was poured out from the prehybridized filters into an alternative 150-mm round plastic container, the remainder of the hybridization buffer mixed with the four labeled probes, and the filters were immersed as described above. The container lid was closed and the hybridization was effected in the Thomastat Shaker T-22S at 32°C and 40 rpm overnight.

The filters placed in another round plastic container with a lid were washed by shaking in the Thomastat Shaker T-22S at 40 rpm with each of the following washes:
1) 2 x SSPE Buffer, 0.1 % SDS 32°C 10 min
2) 2 x SSPE Buffer, 0.1 % SDS 50°C 1.5 hrs
3) 0.5 x SSPE Buffer, 0.1 % SDS 50°C 1.5 hrs

The residual washing buffer was blotted from the filters, and they were allowed to dry at room temperature for at least 1.5 hrs. Twelve filters were arranged in two 35.6 x 43.2-cm Fuji EC-A Cassettes and exposed with Hyperfilm-MP X-ray film (Amersham) for 3 - 4 days.

### <Recovery of Phages>

The X-ray films were developed and the positions of positive plaques were determined according to the orientation marks with the aid of a Bright Light Box for illumination. The 5 x 5-mm top agarose plugs, whose centers coincided with positive signals on the X-ray films, were excised with 21G needles and put into 4-ml sample vials with caps each containing 0.3 ml of SM buffer. Phages were eluted for 1 day at 4°C.

### <Second Screening of Phages>

The second screening of phages was basically the same as described above for the first screening. After preliminary adjustment there was no more need for titration of phage eluate for plating: an amount of phages corresponding to 0.0001 µl of phage eluate per 150-mm Petri dish was found to be appropriate in most cases. The individual plaques were eluted with 0.2 ml of SM buffer for 1 day at 4°C, supplemented with 14 µl of DMSO (dimethyl sulfoxide), and stored at -70°C until use for propagation and purification of phage DNA.

### <Preparation of Phage DNA>

In the morning of the day before the plating stage, a single colony of E. coli XL1-Blue MRF' was picked up from the CG agar plate, inoculated into 3 ml of CG broth (40 Capsules/L of CircleGrow) supplemented with 0.4 % maltose and 25 µg/ml tetracycline, and incubated with shaking at 37°C. In the evening of the same day, 50 µl of the culture was added to 47 ml of the same pre-warmed growth medium and incubated overnight at 37°C with vigorous shaking. In the following morning, the culture was transferred to a sterile 50-ml Polycarbonate Centrifuge Bottle With Cap (Beckman) and spun at 3,000 rpm for 10 min at 4°C using a Beckman JA 20 rotor. The cell pellet obtained was resuspended in 4.5 ml of chilled sterile 10 mM MgSO₄, and then placed on ice.

The 150-mm Petri dishes (Falcon) containing 1.2 % CG agarose GP-36 supplemented with 25 µg/ml tetracycline were used for phage propagation. The Petri dished in which agar was poured were not dried after solidification. They were stored in the refrigerator, and warmed for 1 hr in a constant-temperature room at 37°C before use.

The 50 µl of phage eluate containing approximately 5 x 10⁶ pfu was mixed well with 450 µl of SM buffer and 500 µl of the cell suspension, incubated at 37°C for 15 min, mixed with 6 ml of melted agarose and spread onto a Petri dish. 3 min later, the dishes were inverted and incubated at 37°C for approximately 8 hrs.

To elute the phages, each plate was covered with 8 ml of SM buffer and 200 µl or chloroform, fastened to a Rotary Shaker R-20 mini (Taitec) and shaken at 150 rpm overnight at 4°C.

The phage eluate was transferred into a 15-ml conical centrifuge tube (Greiner) and the plate was washed with additional 2 ml of SM buffer for 1 hr at room temperature. The second eluate was combined with the first one and the tube was spun at 3500 rpm for 10 min (TOMY TS-7 rotor: Low Speed Centrifuge TOMY LC-122).

QIAGEN Lambda Mini Kit (QUIAGEN) was used for purification of the phage DNA. Eight ml of cleared plate lysate was transferred to a new 15-ml centrifuge tube, mixed with 25 µl of Buffer LI (300 mM NaCl, 100 mM Tris-HCl, pH 7.5, 10 mM EDTA, 0.2 mg/ml BSA, 20 mg/ml RNase A and 6 mg/ml DNase I) and incubated at 37°C for 30 min in the Thermo Alumi Bath ALB-120 (Iwaki). The lysate was combined with 1.6 ml of ice-cold Buffer L2 (30 % polyethylene glycol PEG 6000, 3 M NaCl) and incubated on ice for 60 min. The content of the tube was transferred into a 16×76-mm centrifuge tube (Beckman) and centrifuged in the 50 Ti rotor of a L70 Ultracentrifuge (Beckman) for 15 min at 15,000 rpm, at 4°C. The supernate was discarded, and the tube was placed upside down for 1 min to allow the residual fluid to drain. The phage precipitate formed by PEG precipitation carried out in the previous steps was hardly visible as it was clear and distributed over the wall of the tube. Therefore, 0.65 ml of Buffer L3 (100 mM NaCl, 100 mM Tris-HCl, pH 7.5, 25 mM EDTA) was pipetted several times over the wall to ensure complete resuspension of the pellet and the content was transferred into a 2-ml safe-lock tube (Eppendorf). An equal volume of Buffer 4 (4 % sodium dodecyl sulfate) was added to the tube, mixed gently, heated at 70°C for 10 min in the Thermo Alumi Bath ALB-120, then cooled on ice. The 0.65 ml of Buffer L5 (3 M potassium acetate, pH 5.5) were poured into the tube, mixed immediately but gently and spun at 4°C for 30 min at 15,000 rpm (High Speed Micro Refrigerated Centrifuge MRX-150 (TOMY)). The supernate was transferred to a new 2-ml tube and centrifuged again at room temperature for 10 min at 15,000 rpm to remove any residual suspended or particulate materials.

While the centrifugation was going on, a QIAGEN-tip 20 was placed in a QIAtrack 1 over the waste tray, and the column was equilibrated with 1 ml of Buffer QBT (750 mM NaCl, 50 mM MOPS, pH 7.0, 15 % ethanol, 0.15 % Triton X-100). The QIAGEN-tip was allowed to drain completely. QIAGEN-tip could be left unattended since the resin bed retained some buffer and would not readily dry out.

The supernate was promptly applied onto the QIAGEN-tip and allowed to flow through the resin by gravity. The tip was washed twice with 1 ml of Buffer QC (1.0 M NaCl, 50 mM MOPS, pH 7.0, 15 % ethanol). The upper part of a QIArack 1 was placed over the lower rack fitted with clean 1.5-ml tubes and the DNA was eluted twice with 0.75 ml of Buffer QC (1.25 M NaCl, 50 mM Tris-HCl, pH 8.5, 15 % ethanol) into two alternating 1.5-ml micro centrifuge tubes (Treff Lab).

The DNA was precipitated with 0.7 volumes of isopropanol and centrifuged at room temperature for 30 min at 15,000 rpm. The supernate was carefully removed and discarded. The DNA pellet was briefly washed with 0.5 ml of 70 % ethanol at roomtemperature, and then recentrifuged. The wash with 70 % ethanol at roomtemperature was repeated. Ethanol was completely removed. The pellet was briefly air-dried for 5 min, the DNA in each tube was dissolved in 18 µl of NaOH (pH 8) and the content of two tubes containing the same DNA were combined.

### <Recloning of Phage Insertions>

To determine the sequences of the phage insertions, the DNA fragments from appropriate clones were recloned into pUC18 vector.

The 36 µl of λ DNA solution was mixed with 4.5 µl of 10×H buffer and 4.5 µl of EcoRI (10 U/µl) (Takara). Digestion proceeded overnight at 37°C, then the mixture was distributed among three wells and electrophoresed using 1 % Agarose GP-36 gel at 50 V for 50 min. The insertions were purified with QIAEX II Gel Extraction Kit as described under "Purification of PCR Fragments".

Five µl of eluted fragment was mixed with 15 µl of water and added to the tube of Ready-To-Go pUC18 EcoRI/BAP+Ligase (Pharmacia Biotech). The mixture was incubated at room temperature for 3 min, then at 16°C for 0.5 - 3 hrs in a MIR-153 incubator (Sanyo). The 0.5 µl of the ligation reaction mixture was used for transformation of 25 µl DH5 Competent High Cells (Toyobo). Transformation were accomplished basically according to "Cloning of Purified PCR Fragments" protocol. One tenth of transformants were spread on plates containing 1.4 % Bacto-Agar (Difco), 40 capsules/L CircleGrow (Bio 101, Inc.) and 100 µg/ml ampicillin. The plates were incubated overnight at 37°C.

Three clones from each transformation were picked up and grown at 37°C for 16 - 72 hrs in 2.5 ml of CircleGrow medium supplemented with 100 µg/ml ampicillin. Plasmid DNAs were purified with RPM AFS Kit as described under "Purification of Plasmid DNAs".

The presence of the insertion and the approximate concentration of the purified DNA were estimated by digesting 2 µl of the DNA solution with 2 U EcoRI and then carrying out electrophoresis with 1% agarose gel . The orientation of insertion was determined by DNA sequencing according to the above protocol using M13 Universal Sequencing Primer and M13 Reverse Sequence Primer.

### <Construction of Nested Deletions>

Controlled digestion of the DNA with exonuclease III was employed for constructing unidirectional deletion in double-stranded DNA for sequencing of the recloned DNA insertions. This enzyme is 3'-exonuclease active only on double-stranded DNA: blunt and 5'-overhanging ends are susceptible to digestion, while 3'-overhanging ends of three basis or longer are resistant to the enzyme. In addition, since phosphorothioate bond in the phosphate backbone are resistant to digestion with exonuclease III, recessed 3'-ends (5'-overhangs) which have been "filled-in" with thionucleotides are also resistant to digestion. At least two independent clones carrying each DNA region of the gene were used with Double-Stranded Nested Deletion Kit (Pharmacia Biotech) to create deletions from opposite ends.

Appropriate restriction endonucleases must not cut the DNA insertions. If a suitable pair of restriction enzymes producing 3'-and 5'-overhangs or blunt-end could be utilized, then 2 µg of the purified plasmid DNA was digested with these enzymes for at least 3 hrs. Aliquots of 2 µl each were used to monitor the progress of the digestion by agarose gel electrophoresis. When both digestions were complete, the DNA sample was heated for 10 min at 70°C to inactivate the enzymes.

The end-protection by thionucleotides was selected when no appropriate pair of restriction enzymes could be found and neither endonuclease produced 3'-overhanging ends. In such a case, 2 µg of the plasmid DNA solution were digested with first restriction endonuclease in a volume of 10 µl. Up to 3 µl of this reaction mixture were used to monitor the progress of the reaction. Diluted Klenow fragment (0.05 units/ µl) was prepared by mixing FPLCpure Klenow Fragment (1 unit/µl) with 20 µl of 1 × Klenow Buffer (50 mM Tris-HCl, pH 7.5; 10 mM MgCl₂ and 0.1 mM DTT). The following was added to a 1.5-ml microcentrifuge tube: 7 µl 1 of restriction-digested DNA, 1 µl of 10×Klenow Buffer, 1 µl of dNTP α S Mix (aqueous solution containing 400 µM each of dATP α S, dCTP αS, dGTP α S and dTTP α S) and 1 µl of Diluted Klenow Fragment. The tube was mixed gently, briefly centrifuged and incubated at 37°C for 15 min. The reaction mixture was heated at 65°C for 20 min followed by addition of 20 µl NaCl/glycogen (aqueous solution containing 250 mM NaCl and 250 ng/µl glycogen) and 75 µl of ethanol. The mixture was placed on dry ice for 10 min or cooled at -80°C for at least 1 hr. The precipitated DNA was collected by centrifugation at 4°C for 10 min at 15,000 rpm. The supernate was carefully removed and discarded. The pellet was rinsed with 250 µl of 70 % ethanol and centrifuged for 1 min. The supernate was carefully removed. The pellet was dried under vacuum for 5 min and redissolved in 10 µl of water. The DNA was digested with the second restriction enzyme in a final reaction volume of 20 µl and heated for 10 min at 70°C to inactivate the enzyme.

In a microcentrifuge tube, a S1 nuclease/buffer mixture was prepared as follows: 33 µl of S1 Buffer, 66 µl of distilled water and 1 µl of S1 nuclease. Three µl of this mixture was pipetted into each of 20 microcentrifuge tubes, and placed on ice.

The 24 µl of 2XExoIII buffer of appropriate NaCl concentration was prepared. The concentration of NaCl should be 75 mM or lower after dilution with an equal volume of the double-digested DNA.

In a 1.5-ml microcentrifuge tube, 20 µl of 2×ExoIII buffer were mixed with 20 µl (2 µg) of the double-digested DNA, and equilibrated at 30°C for 2 - 3 min in the Thermo Alumi Bath ALB-120. An aliquot of 2 µl was removed as the "time = 0" control sample, and placed in an appropriate tube containing 3 µl of S1 nuclease/buffer, mixed well and then put on ice.

One µl of exonuclease III was added, mixed gently, and incubation was continued at 30°C. A 2-µl sample was removed every 5 min from the reaction mixture, immediately and thoroughly mixed with 3 µl of S1 nuclease/buffer. All these tubes were kept on ice until all the timed samples were removed from the exonuclease III reaction mixture.

After all of the timed samples were taken and mixed with S1 nuclease/buffer, they were incubated simultaneously at room temperature for 30 min.

One µl of S1 Nuclease Stop Solution was added to each of the samples, and the tubes were incubated at 65°C for 30 min. Half of each timed sample was used for electrophoretic analysis of deletions, and the other half was used for recircularization by ligation. These two processes were carried out simultaneously for all samples, and the results of the deletion analysis were then used to select recircularized samples to be used for transformation.

Three µl of each sample was mixed with 2 µl of Loading Buffer (50 % glycerol, 1 mM EDTA and 0.01 % bromphenol blue) and analyzed by 30-min electrophoresis on a 1 % agarose gel (Agarose GP-36 from Nacalai Tesque) containing 0.5 µg/ml ethidium bromide.

While the electrophoresis was in progress, the remaining 3 µl of each timed sample was used for re-ligation. A ligation mixture was prepared by mixing the following materials in a 1.5-ml microcentrifuge tube: 40 µl of 10 × Ligation Buffer, 80 µl of 25 % PEG, 2 µl of T4 DNA ligase and 218 µl of distilled water. Seventeen µl of this ligation mixtures was added to the 3 µl of each timed sample, mixed gently and incubated at room temperature for 2 hrs.

When the electrophoresis was completed, the gel was inspected to determine which timed samples contained deletions of interest. Just those samples that contained deletions of interest were used to transform E. coli cells. 2 µl 1 of ligation reaction mixture was employed for transformation of 10 µl of DH5 Competent High Cells (Toyobo). Transformations were accomplished according to "Cloning of Purified PCR Fragments" protocol. One tenth of transformants were spread on the plate containing 1.4 % Bacto-Agar (Difco), 40 capsules/L of CircleGrow (Bio 101, Inc.) and 100 µl/ml ampicillin. Two colonies from each plate were grown and analyzed for the extent of deletions by electrophoresis of the plasmid DNAs digested with EcoRI followed by DNA sequencing as described above. The DNA sequences were linked with DNASIS computer program to compile the full-length insertion.

### <Computer Analysis of Sequencing Data>

The analysis of nucleotide sequences was performed using DNASIS computer program and some software available through the Internet: BLAST (Basic Local Alignment Search Tool), MOTIF (Protein Sequence Motif Search), PSORT (Prediction of Protein Sorting Signals and Localization Sites in Amino Acid Sequences), SOSUI (Prediction of Transmembrane Segments), SIM (Alignment Tool for protein sequences) and GeneStream align.

### <Northern Hybridization>

Multiple Tissue Northern (MTN) Blots (Clontech) containing approximately 2 µg of polyA⁺RNA per lane from different human cells were hybridized with hGHITS-specific probes as described above under "Plaque Hybridization".

Prehybridization and hybridization were performed at 42°C rather than 32°C. The following washes were carried out:
1) 2×SSPE Buffer, 0.1 % SDS 42°C 10 min
2) 2×SSPE Buffer, 0.1 % SDS 65°C 1.5 hrs
3) 0.5×SSPE Buffer, 0.1 % SDS 65°C 1.5 hrs
4) 0.1×SSPE Buffer, 0.1 % SDS 65°C 1.5 hrs
5) 0.1×SSPE Buffer, 0.1 % SDS 65°C 1.5 hrs

BIOMAX MS scientific imaging film (Kodak) was employed since it is nearly eight times more sensitive than Hyperfilm-MP. The film was exposed to the membranes using Fuji EC-A Cassette with intensifying screen at -80°C for 3 weeks. Before the development of the film, the cassette was warmed for at least 1 hr at room temperature.

## Claims

1. A DNA having a nucleotide sequence set forth under SEQ ID NO:1 or SEQ ID NO:2 in the Sequence Listing.

2. A DNA composing a human gene whose expression is inhibited by a growth factor and which has a nucleotide sequence set forth under SEQ ID NO:1 or SEQ ID NO:2 in the Sequence Listing or a nucleotide sequence with deletion, substitution or addition of one or more bases relative to either of said nucleotide sequences.

3. The DNA composing a human gene of claim 2, wherein the growth factor is human growth hormone or insulin-like growth factor-1.

4. A DNA composing a human gene coding for a tumor suppressive protein which has a nucleotide sequence set forth under SEQ ID NO:1 or SEQ ID NO:2 in the Sequence Listing or a nucleotide sequence with deletion, substitution or addition of one or more bases relative to either of said nucleotide sequences.

5. A protein having an amino acid sequence set forth under SEQ ID NO:3 or SEQ ID NO:4 in the Sequence Listing.

6. A protein whose expression is inhibited by a growth factor and which has an amino acid sequence set forth under SEQ ID NO:3 or SEQ ID NO:4 in the Sequence Listing or an amino acid sequence with deletion, substitution or addition of one or more amino acids relative to either of said amino acid sequences.

7. The protein of claim 6 wherein the growth factor is human growth hormone or insulin-like growth factor-1.

8. A tumor suppressive protein having an amino acid sequence set forth under SEQ ID NO:3 or SEQ ID NO:4 in the Sequence Listing or an amino acid sequence with deletion, substitution or addition of one or more amino acids relative to either of said amino acid sequences.

9. A DNA comprising a nucleotide sequence coding a protein having an amino acid sequence recited in one of claims 5 to 8.

10. A probe comprising a DNA which hybridizes with a DNA consisting of a nucleotide sequence of claim 1, 2. 3 or 4.

11. A probe comprising a DNA which hybridizes with a DNA consisting of a nucleotide sequence of claim 9.

12. A recombinant vector including a DNA consisting of a nucleotide sequence of claim 1, 2, 3 or 4.

13. A recombinant vector including a DNA consisting of a nucleotide sequence of claim 9.

14. A DNA fragment for use as a primer and consisting of a partial sequence of a nucleotide sequence of claim 1, 2, 3 or 4.

15. A DNA fragment for use as a primer and consisting of a partial sequence of a nucleotide sequence of claim 9.

16. A diagnostic pharmaceutical preparation for human use comprising a probe of claim 10.

17. A diagnostic pharmaceutical preparation for human use comprising a probe of claim 11.

18. The diagnostic pharmaceutical preparation of claim 16 for examining expression of a tumor suppressor gene which is inhibited by a growth factor, in dwarfism, gigantism, acromegaly, angiopathy, diabetic nephropathy or cardiopathy or in malignant tumor including breast cancer, renal adenocarcinoma, colorectal cancer and leukemia.

19. The diagnostic pharmaceutical preparation of claim 17 for examining expression of a tumor suppressor gene which is inhibited by a growth factor, in dwarfism, gigantism, acromegaly, angiopathy, diabetic nephropathy or cardiopathy or in malignant tumor including breast cancer, renal adenocarcinoma, colorectal cancer and leukemia.

20. An antineoplastic pharmaceutical preparation containing the protein of one of claims 5 to 8.

21. A polyclonal or monoclonal antibody directed against the protein of one of claims 5 to 8.

22. A diagnostic pharmaceutical preparation containing the antibody of claim 21 for examining expression of a tumor suppressor gene.
